(19) Europäisches Patentamt
European Patent Office
Office européen des brevets

(11) Veröffentlichungsnummer: **0 611 776 A2**

(12) # EUROPÄISCHE PATENTANMELDUNG

(21) Anmeldenummer: **94101544.8**

(22) Anmeldetag: **02.02.94**

(51) Int. Cl.5: **C07K 5/08**, C07K 5/10, A61K 37/02

(30) Priorität: **15.02.93 DE 4304453**
**10.03.93 DE 4307438**

(43) Veröffentlichungstag der Anmeldung: **24.08.94 Patentblatt 94/34**

(84) Benannte Vertragsstaaten: **AT BE CH DE DK ES FR GB GR IE IT LI LU MC NL PT SE**

(71) Anmelder: **BAYER AG**

**D-51368 Leverkusen (DE)**

(72) Erfinder: **Häbich, Dieter, Dr.**
**Krummacherstrasse 82**
**D-42115 Wuppertal (DE)**
Erfinder: **Matzke, Michael, Dr.**
**Am Ringelbusch 15**
**D-42113 Wuppertal (DE)**
Erfinder: **Frobel, Klaus, Dr.**
**Paul-Ehrlich-Strasse 9**
**D-42113 Wuppertal (DE)**
Erfinder: **Henkel, Thomas, Dr.**
**Schlieffenstrasse 72**
**D-42329 Wuppertal (DE)**
Erfinder: **Müller, Hartwig, Dr.**
**Wiesenweg 10**
**D-42553 Velbert (DE)**
Erfinder: **Weber, Karl-Heinz, Dr.**
**Auf dem Scheidt 17**
**D-42115 Wuppertal (DE)**
Erfinder: **Reefschläger, Jürgen, Dr.**
**Kranichweg 14**
**D-42111 Wuppertal (DE)**
Erfinder: **Streissle, Gert, Dr.**
**Gellertweg 22**
**D-42115 Wuppertal (DE)**
Erfinder: **Hansen, Jutta, Dr.**
**Claudiusweg 9**
**D-42115 Wuppertal (DE)**
Erfinder: **Neumann, Rainer, Dr.**
**Olefstrasse 11**
**D-50937 Köln (DE)**
Erfinder: **Paessens, Arnold, Dr.**
**Stresemannstrasse 51**
**D-42781 Haan (DE)**

(54) **Neue antiviral wirksame Pseudopeptide.**

(57) Die Erfindung betrifft neue antiviral wirksame Pseudopeptide der allgemeinen Formel (I)

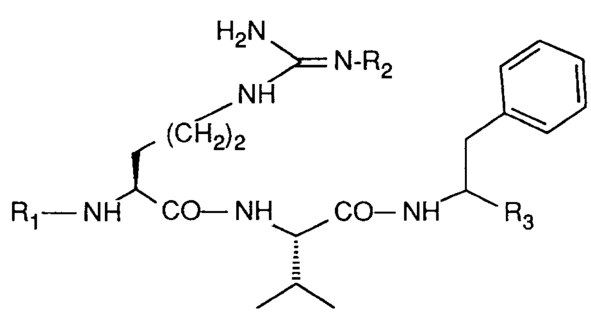

(I)

mit den in der Beschreibung angegebenen Bedeutungen für die Substituenten, ein Verfahren zu ihrer Herstellung sowie ihre Verwendung als antivirale Mittel, insbesondere gegen Cytomegalieviren.

EP 0 611 776 A2

Die vorliegende Erfindung betrifft neue antiviral wirksame Pseudopeptide, ein Verfahren zu ihrer Herstellung sowie ihre Verwendung als antivirale Mittel, insbesondere gegen Cytomegalieviren.

Aus den Publikationen Agric. Biol. Chem. 41, 1313-1314 (1977); 42, 2209 - 2215, (1978) und 43 (2), 243-250, (1979) ist die Isolierung und Charakterisierung des microbiellen alkalischen Proteinase-Inhibitors (MAPI), der durch Streptomyces nigrescens WT-27 produziert wird, bekannt. Ferner ist bekannt, daß MAPI aus der Fermentation von Streptomyces libani S-35 (Bezeichnung Sp. Chymostatin) erhalten werden kann [vgl. Agric. Biol. Chem. 43, 691 (1979)].

In der Publikation J. Antibiot. 44, 1019 (1991) wird für den MAPI eine HIV-1-proteaseinhibitorische Wirkung beschrieben. In der Publikation WO 92/14 696 werden Peptidaldehyde als HIV-Proteaseinhibitoren beschrieben.

Als Verbindungsklassen mit anti-Cytomegalieaktivität sind verschiedene Nucleosid- und Nucleotidanaloga, Anthrachinon-Derivate, Cobalt-Komplexe, Macrolide und Acylpeptide [EP 488 041] bekannt.

Die vorliegende Erfindung betrifft jetzt neue antiviral wirksame Pseudopeptide der allgemeinen Formel (I)

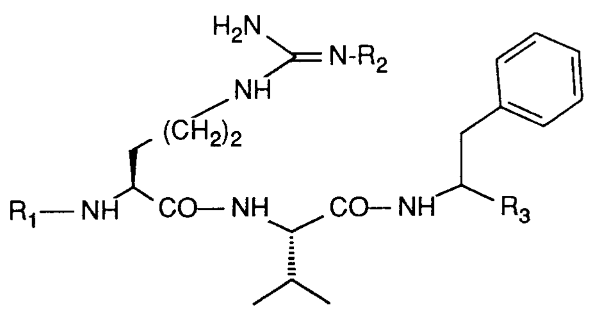

(I)

in welcher

$R^1$ für eine Aminoschutzgruppe steht, oder für einen Rest der Formel $R^4$-NH-CO- steht, worin

$R^4$ Cycloalkyl mit 3 bis 6 Kohlenstoffatomen bedeutet oder geradkettiges oder verzweigtes Alkyl mit bis zu 18 Kohlenstoffatomen bedeutet, das gegebenenfalls durch Hydroxy, geradkettiges oder verzweigtes Alkoxy mit bis zu 4 Kohlenstoffatomen, Halogen, Trifluormethyl, Trifluormethoxy, Cycloalkyl mit 3 bis 6 Kohlenstoffatomen oder durch Aryl mit 6 bis 10 Kohlenstoffatomen substituiert ist, das seinerseits bis zu 2-fach gleich oder verschieden durch Carboxy, Cyano, Hydroxy, Halogen, Perhalogenalkyl mit bis zu 5 Kohlenstoffatomen oder durch geradkettiges oder verzweigtes Acyl, Alkoxy oder Alkoxycarbonyl mit jeweils bis zu 6 Kohlenstoffatomen substituiert sein kann, oder Alkyl gegebenenfalls durch eine Gruppe der Formel $-CO_2R^5$ substituiert ist, worin

$R^5$ Wasserstoff oder geradkettiges oder verzweigtes Alkyl oder Alkenyl mit jeweils bis zu 8 Kohlenstoffatomen bedeutet, die gegebenenfalls durch Phenyl substituiert sind, oder

$R^4$ Aryl mit 6 bis 10 Kohlenstoffatomen bedeutet, das gegebenenfalls bis zu 3-fach gleich oder verschieden durch Carboxy, Halogen, Hydroxy, Cyano, Perhalogenalkyl mit bis zu 5 Kohlenstoffatomen oder durch geradkettiges oder verzweigtes Acyl, Alkoxy, Alkoxycarbonyl oder Vinylalkoxycarbonyl mit jeweils bis zu 6 Kohlenstoffatomen substituiert ist, oder einen Aminosäurerest der Formel

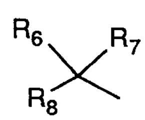

bedeutet, worin

$R^6$ und $R^7$ gleich oder verschieden sind und Wasserstoff oder Methyl bedeuten, oder

$R^6$ und $R^7$ gemeinsam einen 5- oder 6-gliedrigen gesättigten carbocyclischen Ring bilden, oder

$R^6$ Wasserstoff oder Methyl bedeutet
und

$R^7$ Cycloalkyl mit 3 bis 8 Kohlenstoffatomen oder Aryl mit 6 bis 10 Kohlenstoffatomen oder Wasserstoff bedeutet, oder geradkettiges oder verzweigtes Alkyl mit bis zu 8 Kohlenstoffatomen bedeutet,
wobei das Alkyl gegebenenfalls durch Methylthio, Hydroxy, Mercapto, Guanidyl oder durch eine Gruppe der Formel $-NR^9R^{10}$ oder $R^{11}$-OC- substituiert ist,
worin

$R^9$ und $R^{10}$ unabhängig voneinander für Wasserstoff, geradkettiges oder verzweigtes Alkyl mit bis zu 8 Kohlenstoffatomen oder Phenyl stehen,
und

$R^{11}$ Hydroxy, Benzyloxy, Alkoxy mit bis zu 6 Kohlenstoffatomen oder die oben aufgeführte Gruppe $-NR^9R^{10}$ bedeutet,
oder das Alkyl gegebenenfalls durch Cycloalkyl mit 3 bis 8 Kohlenstoffatomen oder durch Aryl mit 6 bis 10 Kohlenstoffatomen substituiert ist, das seinerseits durch Hydroxy, Halogen, Nitro, Alkoxy mit bis zu 8 Kohlenstoffatomen oder durch die Gruppe $-NR^9R^{10}$ substituiert ist,
worin

$R^9$ und $R^{10}$ die oben angegebene Bedeutung haben,
oder das Alkyl gegebenenfalls durch einen 5- bis 6-gliedrigen stickstoffhaltigen Heterocyclus oder Indolyl substituiert ist, worin die entsprechenden -NH-Funktionen gegebenenfalls durch Alkyl mit bis zu 6 Kohlenstoffatomen oder durch eine Aminoschutzgruppe geschützt sind,

$R^8$ geradkettiges oder verzweigtes Alkyl mit bis zu 8 Kohlenstoffatomen bedeutet, das gegebenenfalls durch Hydroxy oder geradkettiges oder verzweigtes Alkoxy mit bis zu 6 Kohlenstoffatomen substituiert ist, oder Carboxy, Allyloxycarbonyl, geradkettiges oder verzweigtes Alkoxycarbonyl mit bis zu 8 Kohlenstoffatomen oder Benzyloxycarbonyl bedeutet,

$R^2$ für Wasserstoff, für eine Aminoschutzgruppe oder für einen Rest der Formel $-SO_2-R^{12}$ steht,
worin

$R^{12}$ Methyl oder Phenyl bedeutet, das gegebenenfalls bis zu 4-fach gleich oder verschieden durch Methyl oder Methoxy substitiert ist, oder einen Rest der Formel

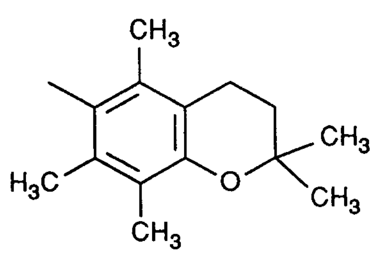

bedeutet,

$R^3$ für Formyl oder Carboxy steht oder
für geradkettiges oder verzweigtes Alkoxycarbonyl mit bis zu 8 Kohlenstoffatomen steht oder
für einen Rest der Formel $-CH_2-OR^{13}$ oder $-CH(OR^{14})_2$ steht,
worin

$R^{13}$ und $R^{14}$ gleich oder verschieden sind und Wasserstoff, geradkettiges oder verzweigtes Alkyl mit bis zu 6 Kohlenstoffatomen oder eine Hydroxyschutzgruppe bedeuten,

mit der Maßgabe, daß
$R^4$ nicht den Rest der Formel

$$HO_2C\text{-}CH\text{-}CH_2\text{-}C_6H_5$$

bedeuten darf, wenn $R^2$ für Wasserstoff und $R^3$ entweder für Formyl oder für Carboxy steht, und

$R^4$ nicht den Rest der Formel

$$H_3C\text{-}O_2C\text{-}CH\text{-}CH_2\text{-}C_6H_5$$

bedeuten darf, wenn

$R^2$ für Wasserstoff und $R^3$ für die $-CH(OCH_3)_2$-Gruppe steht,

gegebenenfalls in einer isomeren Form, und deren Salze.

Die erfindungsgemäßen Verbindungen der allgemeinen Formel (I) können auch in Form ihrer Salze vorliegen. Im allgemeinen seien hier Salze mit organischen und anorganischen Basen oder Säuren genannt.

Zu den Säuren, die addiert werden können, gehören vorzugsweise Halogenwasserstoffsäuren, wie z.B. die Fluorwasserstoffsäure, Chlorwasserstoffsäure und die Bromwasserstoffsäure, insbesondere die Fluor- und Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Malonsäure, Oxalsäure, Gluconsäure, Bernsteinsäure, Fumarsäure, Weinsäure, Zitronensäure, Salizylsäure, Sorbinsäure und Milchsäure sowie Sulfonsäuren, wie z.B. p-Toluolsulfonsäure, 1,5-Naphthalindisulfonsäure oder Camphersulfonsäure.

Physiologisch unbedenkliche Salze können ebenso Metall- oder Ammoniumsalze der erfindungsgemäßen Verbindungen sein, welche eine freie Carboxylgruppe besitzen, sein. Besonders bevorzugt sind z.B. Natrium-, Kalium-, Magnesium oder Calciumsalze, sowie Ammoniumsalze, die abgeleitet sind von Ammoniak, oder organischen Aminen wie beispielsweise Ethylamin, Di- bzw. Triethylamin, Di- bzw. Triethanolamin, Dicyclohexylamin, Dimethylaminoethanol, Arginin, Lysin oder Ethylendiamin.

Hydroxyschutzgruppe im Rahmen der oben angegebenen Definition steht im allgemeinen für eine Schutzgruppe aus der Reihe: tert.Butoxydiphenylsilyl, Trimethylsilyl, Triethylsilyl, Triisopropylsilyl, tert.Butyldimethylsilyl, tert.Butyldiphenylsilyl, Triphenylsilyl, Trimethylsilylethoxycarbonyl, Benzyl, Benzyloxycarbonyl, 2-Nitrobenzyl, 4-Nitrobenzyl, 2-Nitrobenzyloxycarbonyl, 4-Nitrobenzyloxycarbonyl, tert Butyloxycarbonyl, Allyloxycarbonyl, 4-Methoxybenzyl, 4-Methoxybenzyloxycarbonyl, Formyl, Acetyl, Trichloracetyl, 2,2,2-Trichlorethoxycarbonyl, 2,4-Dimethoxybenzyl, 2,4-Dimethoxybenzyloxycarbonyl, Methylthiomethyl, Methoxyethoxymethyl, [2-(Trimethylsilyl)ethoxy]methyl, 2-(Methylthiomethoxy)ethoxycarbonyl, Benzoyl, 4-Methylbenzoyl, 4-Nitrobenzoyl, 4-Fluorbenzoyl, 4-Chlorbenzoyl oder 4-Methoxybenzoyl. Bevorzugt sind Acetyl, Benzoyl, Benzyl oder Methylbenzyl.

Aminoschutzgruppe im Rahmen der Erfindung sind die üblichen in der Peptid-Chemie verwendeten Aminoschutzgruppen.

Hierzu gehören bevorzugt: Benzyloxycarbonyl, 3,4-Dimethoxybenzyloxycarbonyl, 3,5-Dimethoxybenzyloxycarbonyl, 2,4-Dimethoxybenzyloxycarbonyl, 4-Methoxybenzyloxycarbonyl, 4-Nitrobenzyloxycarbonyl, 2-Nitrobenzyloxycarbonyl, 2-Nitro-4,5-dimethoxybenzyloxycarbonyl, Methoxycarbonyl, Ethoxycarbonyl, Propoxycarbonyl, Isopropoxycarbonyl, Butoxycarbonyl, Isobutoxycarbonyl, tert.Butoxycarbonyl, Allyloxycarbonyl, Vinyloxycarbonyl, 3,4,5-Trimethoxybenzyloxycarbonyl, Cyclohexoxycarbonyl, 1,1-Dimethylethoxycarbonyl, Adamantylcarbonyl, Phthaloyl, 2,2,2-Trichlorethoxycarbonyl, 2,2,2-Trichlor-tertbutoxycarbonyl, Menthyloxycarbonyl, Phenoxycarbonyl, 4-Nitrophenoxycarbonyl, Fluorenyl-9-methoxycarbonyl, Formyl, Acetyl, Propionyl, Pivaloyl, 2-Chloracetyl, 2-Bromacetyl, 2,2,2-Trifluoracetyl, 2,2,2-Trichloracetyl, Benzoyl, 4-Chlorbenzoyl, 4-Brombenzoyl, 4-Nitrobenzoyl, Phthalimido, Isovaleroyl oder Benzyloxymethylen, 4-Nitrobenzyl, 2,4-Dinitrobenzyl oder 4-Nitrophenyl.

Die erfindungsgemäßen Verbindungen der allgemeinen Formel (I) besitzen, wie der Rest der allgemeinen Formel (II)

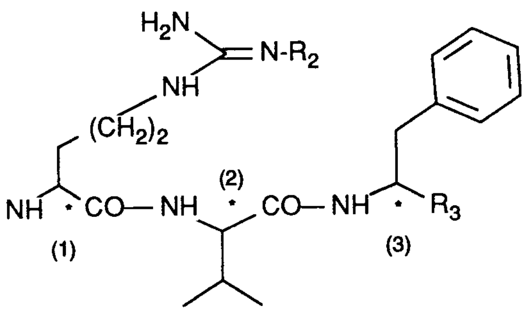

(II)

zeigt, mindestens 3 asymmetrische Kohlenstoffatome (*). Sie können unabhängig voneinander in der D- oder L-Form, und R- oder S-Konfiguration vorliegen. Die Erfindung umfaßt die optischen Antipoden ebenso wie die Isomerengemische oder Racemate.

Die erfindungsgemäßen Verbindungen der allgemeinen Formel (I) können in stereoisomeren Formen existieren, beispielsweise entweder wie Bild und Spiegelbild (Enantiomere), oder die sich nicht wie Bild und Spiegelbild (Diastereomere) verhalten, beziehungsweise als Diastereomerengemisch vorliegen. Die Erfindung betrifft sowohl die Antipoden, Racemformen, Diastereomerengemische sowie die reinen Isomeren. Die Racemformen lassen sich ebenso wie die Diastereomeren in bekannter Weise in die stereoisomer einheitlichen Bestandteile trennen [vgl. E.L. Eliel, Stereochemistry of Carbon Compounds, McGraw Hill, 1962]. Die Trennung in die stereoisomer einheitlichen Verbindungen erfolgt beispielsweise über eine chromatograptusche Racematspaltung von diastereomeren Estern und Amiden oder an optisch aktiven Phasen. Außerdem ist eine Kristallisation von diastereomeren Salzen möglich.

Bevorzugt sind Verbindungen der allgemeinen Formel (I), in welcher

$R^1$ für Acetyl, tert.Butoxycarbonyl (Boc), Benzyloxycarbonyl (Z) oder 9-Fluorenylmethoxycarbonyl (Fmoc) steht, oder
für einen Rest der Formel $R^4$-NH-CO- steht,
worin

$R^4$ Cyclopentyl oder Cyclohexyl bedeutet oder geradkettiges oder verzweigtes Alkyl mit bis zu 16 Kohlenstoffatomen bedeutet, das gegebenenfalls durch Hydroxy, Methoxy, Fluor, Chlor, Brom, Trifluormethyl, Trifluormethoxy, Cyclopentyl, Cyclohexyl oder Phenyl substituiert ist, das seinerseits bis zu 2-fach gleich oder verschieden durch Carboxy, Cyano, Hydroxy, Fluor, Chlor, Brom, Perhalogenalkyl mit bis zu 4 Kohlenstoffatomen oder durch geradkettiges oder verzweigtes Acyl, Alkoxy oder Alkoxycarbonyl mit jeweils bis zu 4 Kohlenstoffatomen substituiert sein kann, oder
Alkyl gegebenenfalls durch eine Gruppe der Formel $-CO_2R^5$ substituiert ist,
worin

$R^5$ Wasserstoff oder geradkettiges oder verzweigtes Alkyl oder Alkenyl mit jeweils bis zu 6 Kohlenstoffatomen bedeutet, die gegebenenfalls durch Phenyl substituiert sind,
oder

$R^4$ Phenyl oder Naphthyl bedeutet, das gegebenenfalls bis zu 3-fach gleich oder verschieden durch Carboxy, Fluor, Chlor, Brom, Hydroxy, Cyano, Perhalogenalkyl mit bis zu 4 Kohlenstoffatomen oder durch geradkettiges oder verzweigtes Acyl, Alkoxy, Alkoxycarbonyl oder Vinylalkoxycarbonyl mit jeweils bis zu 5 Kohlenstoffatomen substituiert ist, oder einen Aminosäurerest der Formel

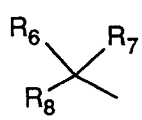

bedeutet,
worin

$R^6$ und $R^7$ gleich oder verschieden sind und Wasserstoff oder Methyl bedeuten, oder

$R^6$ und $R^7$ gemeinsam einen Cyclopentyl- oder Cyclohexylring bilden,
oder

$R^6$ Wasserstoff oder Methyl bedeutet
und

$R^7$ Cyclopentyl, Cyclohexyl, Phenyl oder Wasserstoff bedeutet, oder
geradkettiges oder verzweigtes Alkyl mit bis zu 6 Kohlenstoffatomen bedeutet,
wobei das Alkyl gegebenenfalls durch Methylthio, Hydroxy, Mercapto, Guanidyl, Amino, Carboxy oder $H_2N$-CO-substituiert sein kann,

oder das Alkyl durch Cyclohexyl, Naphthyl oder Phenyl substituiert ist, das seinerseits durch Fluor, Hydroxy, Nitro oder Alkoxy mit bis zu 4 Kohlenstoffatomen substituiert sein kann,

oder das Alkyl gegebenenfalls durch Indolyl, Imidazolyl, Pyridyl, Triazolyl oder Pyrazolyl substituiert ist, wobei die entsprechenden -NH-Funktionen gegebenenfalls durch Alkyl mit bis zu 4 Kohlenstoffatomen oder durch eine Aminoschutzgruppe geschützt sind,

$R^8$ geradkettiges oder verzweigtes Alkyl mit bis zu 6 Kohlenstoffatomen bedeutet, das gegebenenfalls durch Hydroxy oder geradkettiges oder verzweigtes Alkoxy mit bis zu 4 Kohlenstoffatomen substituiert ist, oder
Carboxy, Allyloxycarbonyl, geradkettiges oder verzweigtes Alkoxycarbonyl mit bis zu 6 Kohlenstoffatomen oder Benzyloxycarbonyl bedeutet,

$R^2$ für Wasserstoff, Benzyloxycarbonyl, tert.Butoxycarbonyl oder
für einen Rest der Formel $-SO_2-R^{12}$ steht,
worin

$R^{12}$ Methyl oder Phenyl bedeutet, das gegebenenfalls bis zu 4-fach gleich oder verschieden durch Methyl oder Methoxy substituiert ist, oder einen Rest der Formel

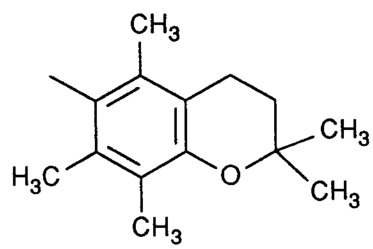

bedeutet,

$R^3$ für Formyl oder Carboxy steht oder
für geradkettiges oder verzweigtes Alkoxycarbonyl mit bis zu 6 Kohlenstoffatomen steht, oder
für einen Rest der Formel $-CH_2-OR^{13}$ oder $-CH(OR^{14})_2$ steht,
worin

$R^{13}$ und $R^{14}$ gleich oder verschieden sind und Wasserstoff, geradkettiges oder verzweigtes Alkyl mit bis zu 4 Kohlenstoffatomen, Acetyl oder Benzyl bedeuten,

mit der Maßgabe, daß
$R^4$ nicht den Rest der Formel

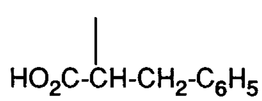

bedeuten darf, wenn $R^2$ für Wasserstoff und $R^3$ entweder für Formyl oder für Carboxy steht,
und

$R^4$ nicht den Rest der Formel

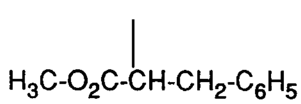

bedeuten darf, wenn

$R^2$ für Wasserstoff und $R^3$ für die $-CH(OCH_3)_2$-Gruppe steht,

gegebenenfalls in einer isomeren Form, und deren Salze.

Besonders bevorzugt sind Verbindungen der allgemeinen Formel (I), in welcher

$R^1$ für Acetyl, tert.Butoxycarbonyl (Boc), Benzyloxycarbonyl (Z) oder 9-Fluorenylmethoxycarbonyl (Fmoc) steht, oder für einen Rest der Formel $R^4$-NH-CO- steht, worin

$R^4$ Cyclopentyl oder Cyclohexyl bedeutet oder geradkettiges oder verzweigtes Alkyl mit bis zu 14 Kohlenstoffatomen bedeutet, das gegebenenfalls durch Hydroxy, Methoxy, Fluor, Trifluormethyl, Trifluormethoxy, Cyclohexyl oder Phenyl substituiert ist, oder das gegebenenfalls durch eine Gruppe der Formel $-CO_2R^5$ substituiert ist, worin

$R^5$ Wasserstoff oder geradkettiges oder verzweigtes Alkyl oder Alkenyl mit jeweils bis zu 4 Kohlenstoffatomen oder Benzyl bedeutet, oder

$R^4$ Phenyl bedeutet, das gegebenenfalls bis zu 2-fach gleich oder verschieden durch Carboxy, Fluor, Hydroxy, Cyano, Trifluormethyl oder durch geradkettiges oder verzweigtes Acyl, Alkoxy, Alkoxycarbonyl oder Vinylalkoxycarbonyl mit jeweils bis zu 4 Kohlenstoffatomen substituiert ist, oder einen Aminosäurerest der Formel

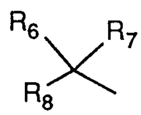

bedeutet, worin

$R^6$ und $R^7$ gleich oder verschieden sind und Wasserstoff oder Methyl bedeuten, oder

$R^6$ und $R^7$ gemeinsam einen Cyclohexylring bilden, oder

$R^6$ Wasserstoff oder Methyl bedeutet und

$R^7$ Cyclopentyl, Cyclohexyl oder Wasserstoff bedeutet, oder geradkettiges oder verzweigtes Alkyl mit bis zu 4 Kohlenstoffatomen bedeutet, wobei das Alkyl gegebenenfalls durch Methylthio, Hydroxy, Mercapto, Guanidyl, Amino, Carboxy oder $H_2N$-CO-substituiert sein kann,

oder das Alkyl durch Cyclohexyl, Naphthyl oder Phenyl substituiert ist, das seinerseits durch Fluor, Chlor oder Alkoxy mit bis zu 4 Kohlenstoffatomen substituiert sein kann,

oder das Alkyl gegebenenfalls durch Imidazolyl, Pyridyl oder Pyrazolyl substituiert ist, wobei die entsprechenden -NH-Funktionen gegebenenfalls durch Methyl, Benzyloxymethylen oder tert.Butyloxycarbonyl (Boc) geschützt sind,

$R^8$ geradkettiges oder verzweigtes Alkyl mit bis zu 4 Kohlenstoffatomen bedeutet, das gegebenenfalls durch Hydroxy oder geradkettiges oder verzweigtes Alkoxy mit bis zu 3 Kohlenstoffatomen substituiert ist, oder Carboxy, Allyloxycarbonyl, geradkettiges oder verzweigtes Alkoxycarbonyl mit bis zu 4 Kohlenstoffatomen oder Benzyloxycarbonyl bedeutet,

$R^2$ für Wasserstoff, oder für einen Rest der Formel $-SO_2-R^{12}$ steht, worin

$R^{12}$ Methyl oder Phenyl bedeutet, das gegebenenfalls bis zu 4-fach gleich oder verschieden durch Methyl oder Methoxy substitiert ist, oder einen Rest der Formel

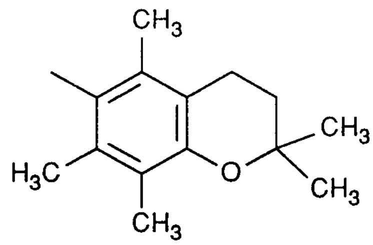

bedeutet,

$R^3$ für Formyl oder Carboxy steht oder für geradkettiges oder verzweigtes Alkoxycarbonyl mit bis zu 4 Kohlenstoffatomen steht, oder für einen Rest der Formel $-CH_2-OR^{13}$ oder $-CH(OR^{14})_2$ steht, worin

$R^{13}$ und $R^{14}$ gleich oder verschieden sind und Wasserstoff, geradkettiges oder verzweigtes Alkyl mit bis zu 3 Kohlenstoffatomen oder Benzyl bedeuten,

mit der Maßgabe, daß

$R^4$ nicht den Rest der Formel

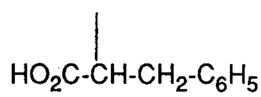

bedeuten darf, wenn $R^2$ für Wasserstoff und $R^3$ entweder für Formyl oder für Carboxy steht,

und

$R^4$ nicht den Rest der Formel

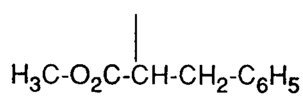

bedeuten darf, wenn

$R^2$ für Wasserstoff und $R^3$ für die $-CH(OCH_3)_2$-Gruppe steht,

gegebenenfalls in einer isomeren Form, und deren Salze.

Außerdem wurde ein neues Verfahren zur Herstellung der erfindungsgemäßen Verbindungen der allgemeinen Formel (I) gefunden, dadurch gekennzeichnet, daß man

Verbindungen der allgemeinen Formel (III)

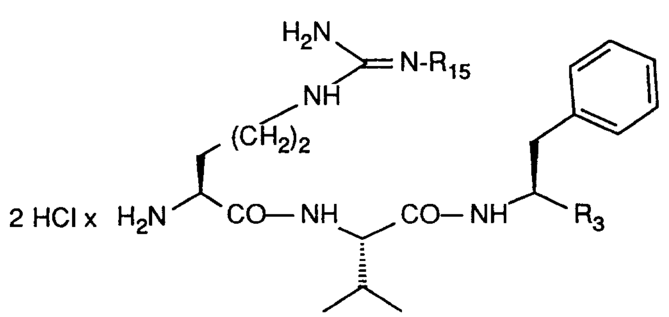

(III)

in welcher

$R^3$ die oben angegebene Bedeutung hat

und

$R^{15}$ die oben angegebenen Bedeutung von $R^2$ hat, aber nicht für Wasserstoff steht

zunächst durch Umsetzung mit Verbindungen der allgemeinen Formel (IV)

$R^4$-N = C = O (IV)

in welcher
$R^4$ die oben angegebene Bedeutung hat,
in inerten Lösemitteln, in Anwesenheit einer Base, in die Verbindungen der allgemeinen Formel (V)

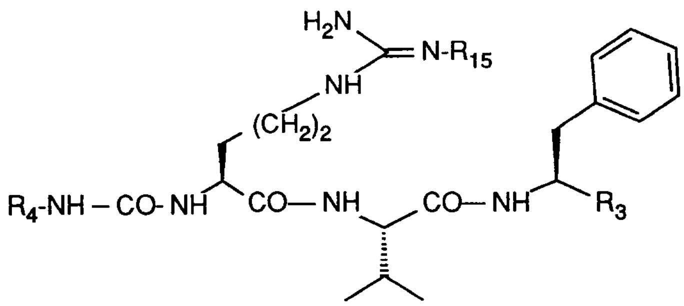

(V)

in welcher
$R^3$, $R^4$ und $R^{15}$ die oben angegebene Bedeutung haben,
überführt,
und im Fall, $R^3$ = $CH_2$-OH, die Verbindungen der allgemeinen Formel (V) ($R^3$ = $CO_2CH_3$) nach üblichen Methoden, vorzugsweise aber mit Natriumborhydrid umsetzt,
und im Fall, $R^3$ = CHO, die Verbindungen der allgemeinen Formel (V), ausgehend von der Hydroxymethylverbindung ($R^3$ = $CH_2$-OH) einer Swern-Oxidation unterzieht,
in Abhängigkeit des Restes $R^{15}$, beispielsweise mit Fluorwasserstoffsäure oder Trifluoressigsäure umsetzt,
und im Fall einer Aminoschutzgruppe ($R^1$/$R^{15}$) diese nach denen in der Peptidchemie üblichen Methoden abspaltet,
und im Fall der Säuren die Ester verseift.

Das erfindungsgemäße Verfahren kann durch folgende Formelschema beispielhaft erläutert werden (Schemen 1 - 4):

Schema 1:

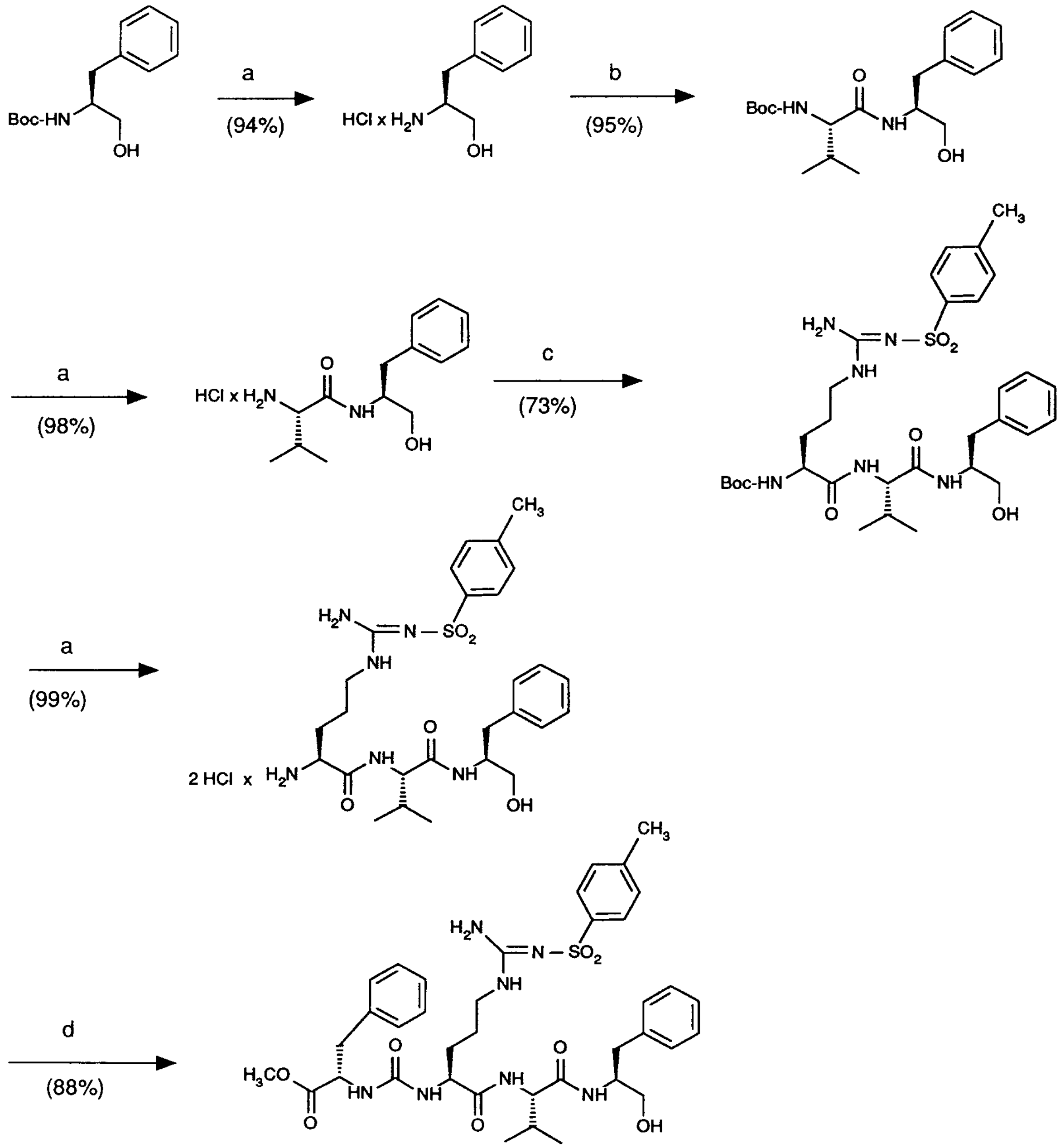

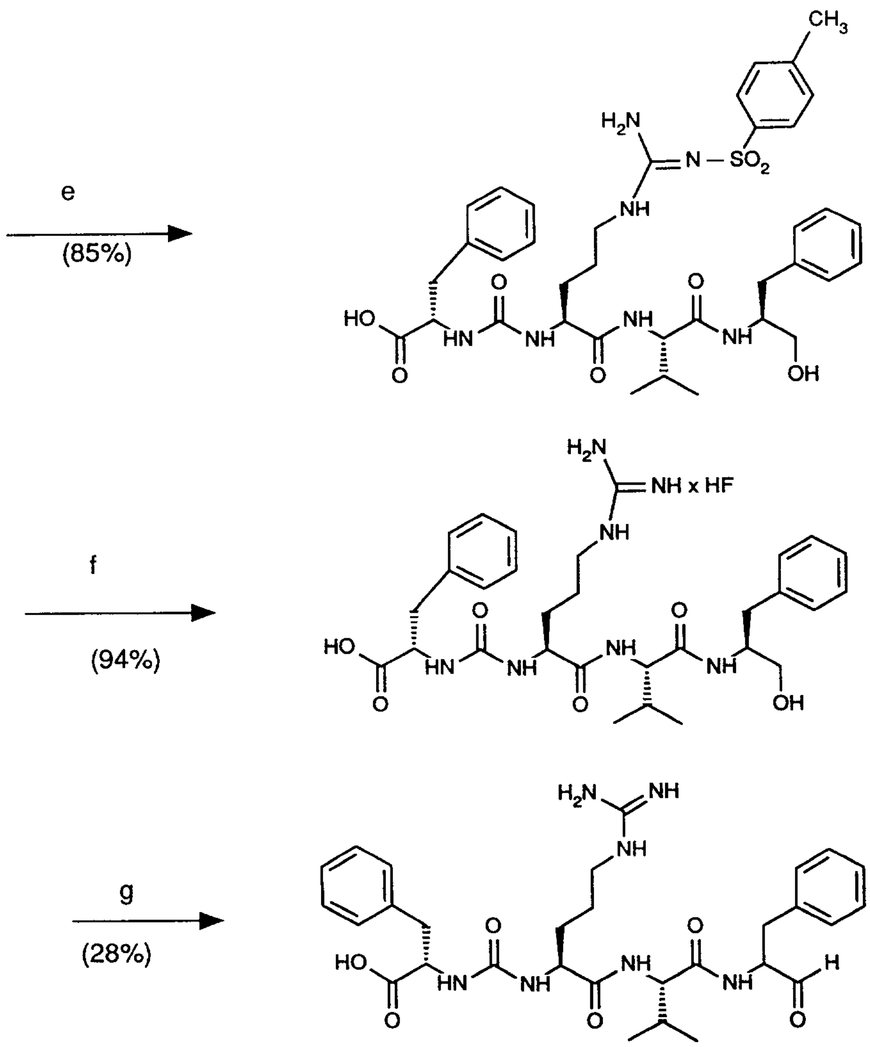


Reagenzien:

a) 4 N HCl in Dioxan; 30 min. Raumtemperatur
b) Boc-Val-OH, HOBT, DCC, $CH_2Cl_2$; 2 h Raumtemperatur
c) Boc-Arg (Tos)-OH, HOBT, DCC, $CH_2Cl_2$/DMF; 1 h Raumtemperatur

d) 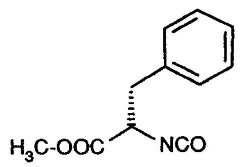 , $NEt_3$, $CH_2Cl_2$; 30 min. Raumtemperatur

e) LiOH, $H_2O$, THF; 20 min. 0°C
f) HF (wasserfrei), p-Kresol; 30 min, 0°C
g) $PyrxSO_3$, $NEt_3$, DMSO; 1 hRaumtemperatur

Schema 2:

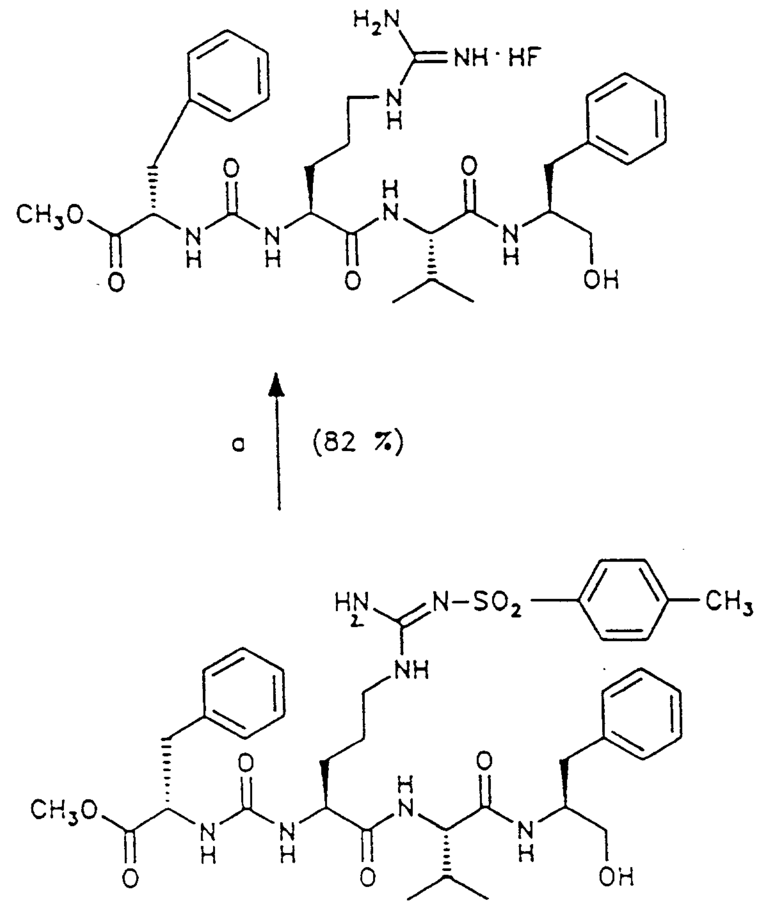

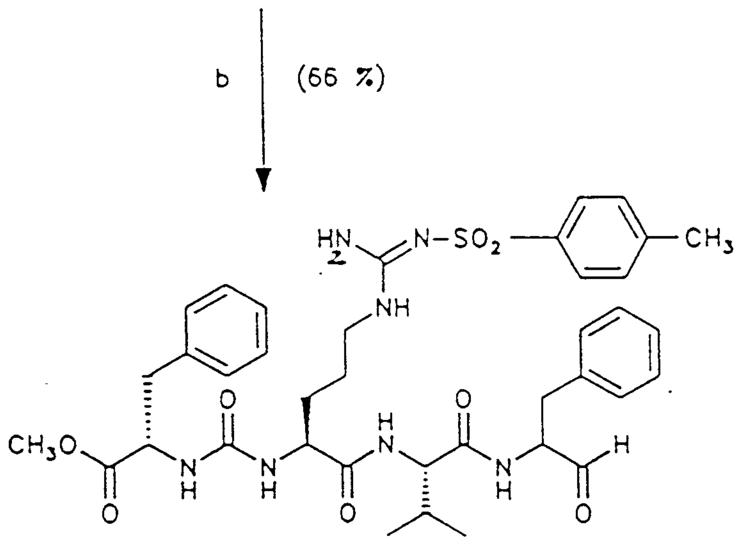


Reogenzien:

a: HF (wasserfrei), PKresol; 30 min. 0°C

b: Pyr SO3, NEt3, DMSO; 1h, Raumtemp.

Schema 3:

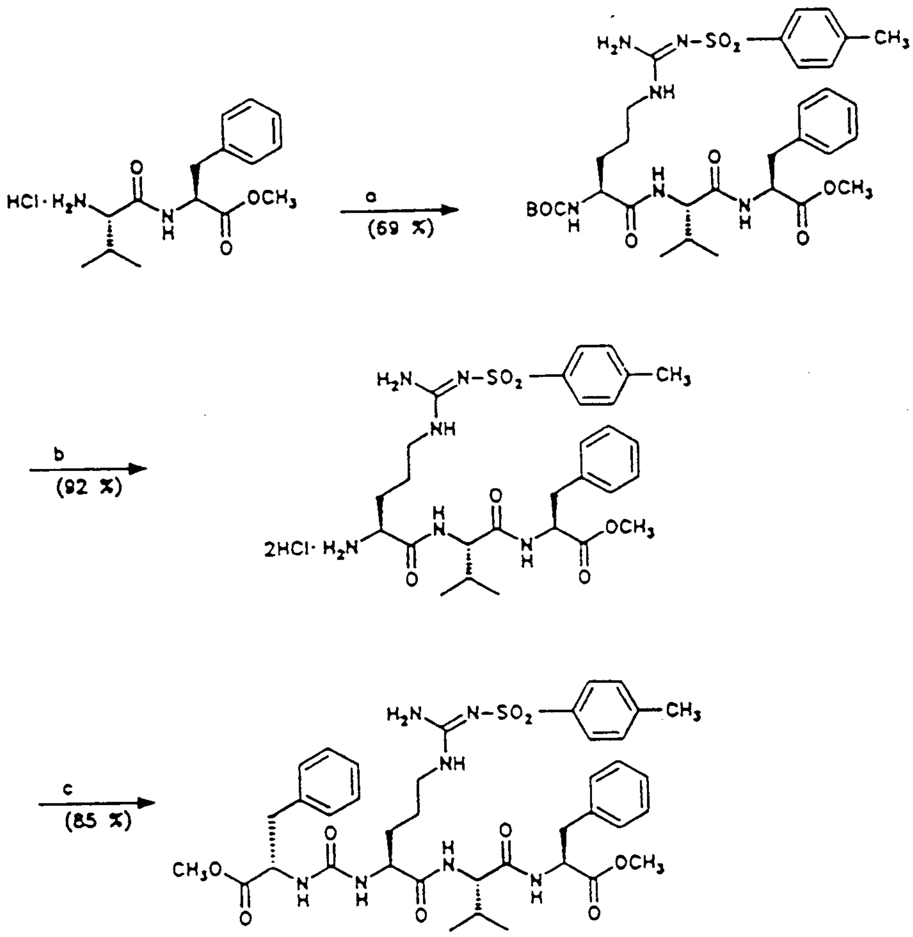

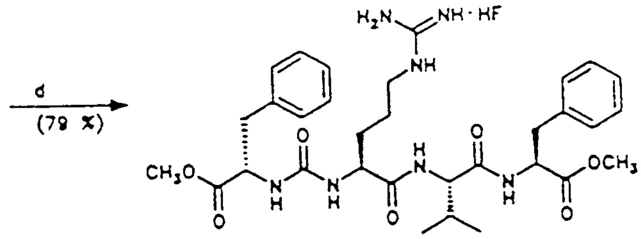


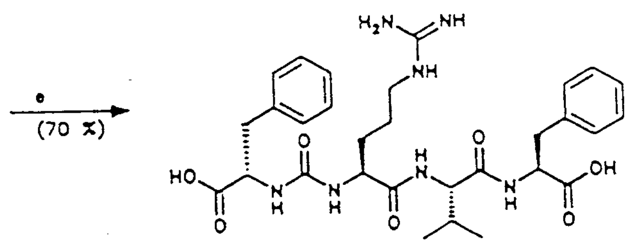


Reagenzien:

a. Boc–Arg(Tos)–OH, HOBT, DCC, $CH_2Cl_2$, DMF; 1 h Raumtemperatur

b. 4N HCl in Dioxan; 30 min., Raumtemperatur

c. 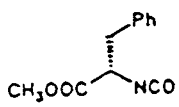 , $NEt_3$, $CH_2Cl_2$; 30 min., Raumtemperatur

d. HF (wasserfrei), p–Kresol; 30 min., 0°C

e. LiOH, $H_2O$, THF; 20 min., 0°C

Schema 4

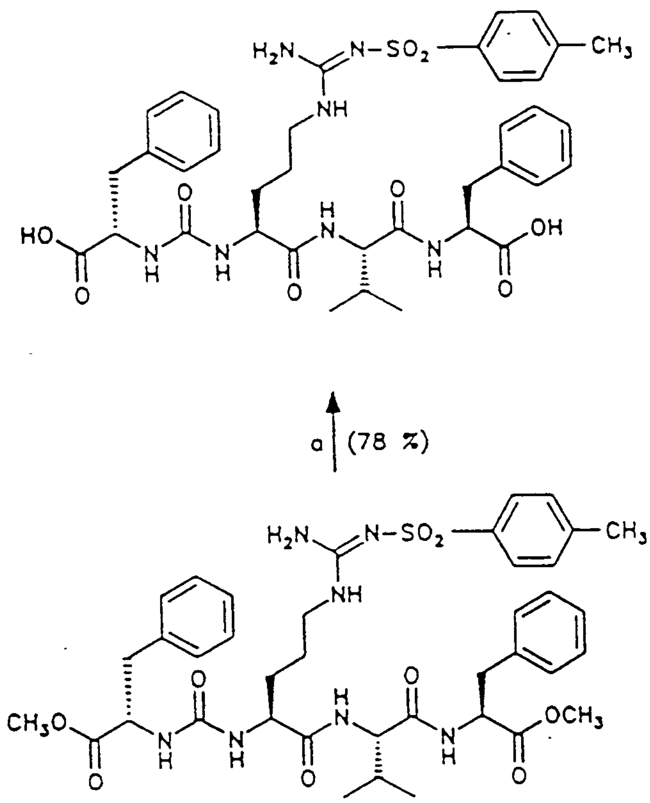

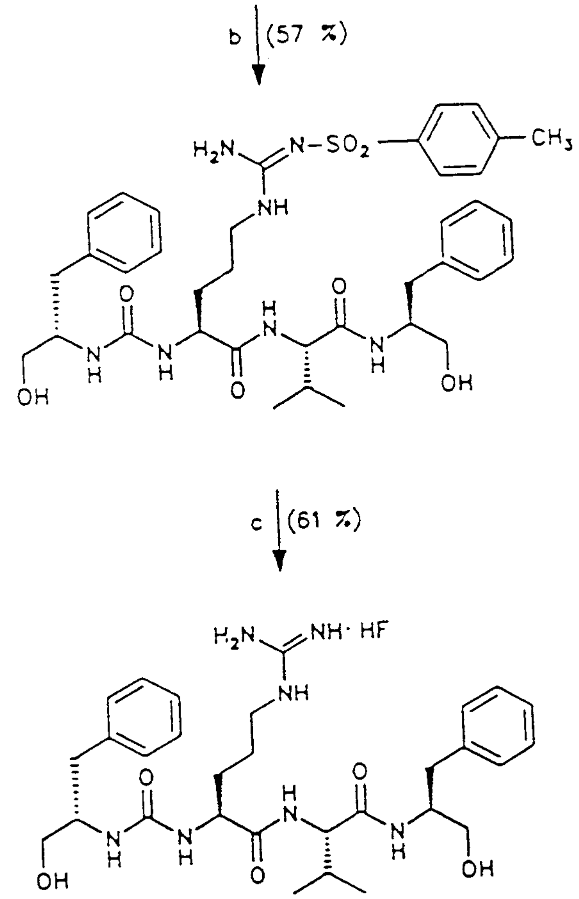


Reagenzien:

a. LiOH, $H_2O$, THF; 20 min., 0°C

b. $NaBH_4$, LiI, THF, MeOH; 5h, 40°C

c. HF (wasserfrei), p-Kresol; 30 min., 0°C

Als Lösemittel eignen sich für alle Verfahrensschritte die üblichen inerten Lösemittel, die sich unter den Reaktionsbedingungen nicht verändern. Hierzu gehören bevorzugt organische Lösemittel wie Ether z.B. Diethylether, Glykolmono- oder -dimethylether, Dioxan oder Tetrahydrofuran, oder Kohlenwasserstoffe wie Benzol, p-Kresol, Toluol, Xylol, Cyclohexan oder Erdölfraktionen oder Halogenkohlenwasserstoffe wie Methylenchlorid, Chloroform, Tetrachlorkohlenstoff, oder Dimethylsulfoxid, Dimethylformamid, Hexamethylphosphorsäuretriamid, Essigester, Pyridin, Triethylamin oder Picolin. Ebenso ist es möglich, Gemische der genannten Lösemittel, gegebenenfalls auch mit Wasser zu verwenden. Besonders bevorzugt sind Methylenchlorid, Tetrahydrofuran, Dioxan und Dioxan/Wasser.

Als Basen eignen sich organische Amine wie (Trialkyl($C_1$-$C_6$)amine wie beispielsweise Triethylamin oder Heterocyclen wie Pyridin, Methylpiperidin, Piperidin oder N-Methylmorpholin. Bevorzugt sind Triethylamin und N-Methylmorpholin.

Die Basen werden im allgemeinen in einer Menge von 0,1 mol bis 5 mol, bevorzugt von 1 mol bis 3 mol jeweils bezogen auf 1 mol der Verbindungen der allgemeinen Formel (III) eingesetzt.

Die Umsetzungen können bei Normaldruck, aber auch bei erhöhtem oder erniedrigtem Druck (z.B. 0,5 bis 3 bar) durchgeführt werden. Im allgemeinen arbeitet man bei Normaldruck.

Die Reaktionen werden in einem Temperaturbereich von 0 ° C bis 100 ° C, vorzugsweise bei 0 ° C bis 30 ° C und bei Normaldruck durchgeführt.

Die Abspaltung der Aminoschutzgruppen erfolgt in an sich bekannter Weise.

Die Abspaltung der Tosylgruppe erfolgt im allgemeinen mit Fluorwasserstoffsäure (wasserfrei) in Gegenwart eines Scavengers, vorzugsweise p-Kresol oder mit Pyridiniumhydrofluorid [s. Matsuura et al.,

J.C.S. Chem. Comm. (1976), 451], in einem Temperaturbereich von -10°C bis +30°C, vorzugsweise bei 0°C.

Die Verseifung der Carbonsäureester erfolgt nach üblichen Methoden, indem man die Ester in inerten Lösemitteln mit üblichen Basen behandelt, wobei die zunächst entstehenden Salze durch Behandeln mit Säure in die freien Carbonsäuren überführt werden können.

Als Basen eignen sich für die Verseifung die üblichen anorganischen Basen. Hierzu gehören bevorzugt Alkalihydroxide oder Erdalkalihydroxide wie beispielsweise Natriumhydroxid, Lithiumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriumethanolat, Natriummethanolat. Kaliumethanolat, Kaliummethanolat oder Kalium- tert.butanolat. Besonders bevorzugt wird Natriumhydroxid oder Lithiumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen. Bevorzugt ist Wasser / Tetrahydrofuran.

Die Verseifung wird im allgemeinen in einem Temperaturbereich von 0°C bis +100°C, bevorzugt von 0°C bis +40°C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base oder die Säure im allgemeinen in einer Menge von 1 bis 3 mol, bevorzugt von 1 bis 1,5 mol bezogen auf 1 mol des Esters eingesetzt. Besonders bevorzugt verwendet man molare Mengen der Reaktanden.

Bei der Durchführung der Reaktion entstehen im ersten Schritt die Salze der erfindungsgemäßen Verbindungen als Zwischenprodukte, die isoliert werden können. Die erfindungsgemäßen Säuren erhält man durch Behandeln der Salze mit üblichen anorganischen Säuren. Hierzu gehören bevorzugt Mineralsäuren wie beispielsweise Chlorwasserstoffsäure, Bromwasserstoffsäure, Schwefelsäure, Zitronensäure oder Phosphorsäure. Es hat sich bei der Herstellung der Carbonsäuren als vorteilhaft erwiesen, die basische Reaktionsmischung der Verseifung in einem zweiten Schritt ohne Isolierung der Salze anzusäuern. Die Säuren können dann in üblicher Weise isoliert werden.

Die Reduktionen können im allgemeinen durch Wasserstoff in Wasser oder in inerten organischen Lösemitteln wie Alkoholen, Ethern oder Halogenkohlenwasserstoffen, oder deren Gemischen, mit Katalysatoren wie Raney-Nickel, Palladium, Palladium auf Tierkohle oder Platin, oder mit Hydriden oder Boranen in inerten Lösemitteln, gegebenenfalls in Anwesenheit eines Katalysators durchgeführt werden.

Bevorzugt wird die Reduktion mit Hydriden, wie komplexen Borhydriden oder Aluminiumhydriden durchgeführt. Besonders bevorzugt wird hierbei Natriumborhydrid, Lithiumaluminiumhydrid oder Natriumcyanoborhydrid eingesetzt.

Als Lösemittel eignen sich hierbei alle inerten organischen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, Tetrahydrofuran, Glykoldimethylether, oder Diethylenglykoldimethylether oder Amide wie Hexamethylphosphorsäuretriamid oder Dimethylformamid, oder Essigsäure. Ebenso ist es möglich, Gemische der genannten Lösemittel zu verwenden. Bevorzugt sind Methanol und Tetrahydrofuran.

Als Katalysatoren können bei den Reduktionen auch Kalium- oder Lithiumjodid, bevorzugt Lithiumjodid eingesetzt werden.

Der Katalysator wird im allgemeinen in einer Menge von 0,1 mol bis 5 mol, bevorzugt von 1 mol bis 3 mol jeweils bezogen auf 1 mol des zu reduzierenden Esters eingesetzt.

Die Umsetzung kann bei normalem, erhöhtem oder bei erniedrigtem Druck durchgeführt werden (z.B. 0,5 bis 5 bar). Im allgemeinen arbeitet man bei Normaldruck.

Die Reduktionen werden im allgemeinen in einem Temperaturbereich von 0°C bis +60°C, vorzugsweise bei +10°C bis +40°C durchgeführt.

Die Oxidation von Alkoholgruppen zu den entsprechenden Aldehyden erfolgt im allgemeinen in einem der oben aufgeführten Lösemitteln, in Anwesenheit einer der oben aufgeführten Basen mit Oxidationsmitteln, wie beispielsweise Kaliumpermanganat, Brom, Jones-Reagenz, Pyridin-dichromat, Pyridinium-chlorochromat, Pyridin-Schwefeltrioxid-Komplex oder Oxalylchlorid [Swern-Oxidation (ClCOCOCl /DMSO / $CH_2Cl_2$ / $NEt_3$) z.B. nach R.E. Ireland et al., J. Org. Chem. 50, 2199 (1985)]. Bevorzugt erfolgt die Oxidation mit Pyridin-Schwefeltrioxid-Komplex in Dimethylsulfoxid in Anwesenheit von Triethylamin.

Die Oxidation erfolgt im allgemeinen in einem Temperaturbereich von 0°C bis +50°C, vorzugsweise bei Raumtemperatur und Normaldruck.

Die Verbindungen der allgemeinen Formel (III) sind neu und können dann nach den in der Peptid-Chemie üblichen Methoden hergestellt werden, indem man beispielsweise Verbindungen der allgemeinen Formel (VI)

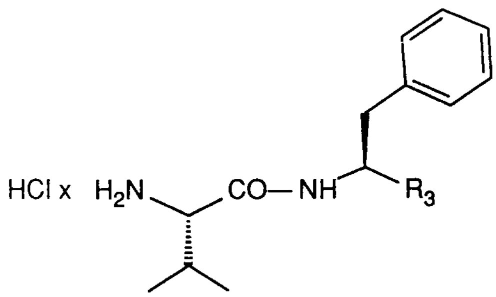

(VI)

in welcher

$R^3$ die oben angegebene Bedeutung hat,

mit den Aminosäurederivaten der Formel (VII)

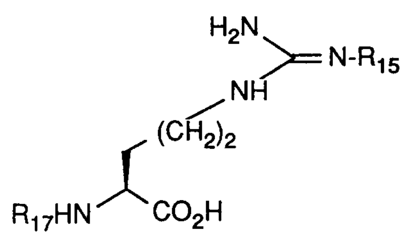

(VII)

in welcher

$R^{15}$ die oben angegebene Bedeutung hat

und

$R^{17}$ für eine der oben aufgeführten Aminoschutzgruppen, vorzugsweise 9-Fluorenylmethoxycarbonyl (Fmoc), tert.Butoxycarbonyl (Boc) oder Benzyloxycarbonyl steht, in einem der oben angegebenen Lösemittel, vorzugsweise Methylenchlorid, in Anwesenheit eines Hilfsstoffes und/oder Base, vorzugsweise HOBT und Dicyclohexylcarbodiimid, umsetzt

und anschließend, ebenfalls nach üblichen Methoden, die Aminoschutzgruppe, vorzugsweise mit Salzsäure in Dioxan abspaltet.

Alle Verfahrensschritte erfolgen bei Normaldruck und in einem Temperaturbereich von 0 °C bis Raumtemperatur, vorzugsweise bei Raumtemperatur.

Die Verbindungen der allgemeinen Formeln (VI) und (VII) sind größtenteils bekannt oder können nach üblichen Methoden hergestellt werden [vgl. J. chem. Res., Synop., (2), 62-63; DE 36 04 510].

Die Verbindungen der allgemeinen Formel (V) sind ebenfalls bekannt [vgl. US 4 929 736].

Die Verbindungen zeigen eine antivirale Wirkung gegenüber Retroviren und Vertretern der Gruppe der Herpetoviridae, besonders gegenüber dem humanen Cytomegalovirus (HCMV).

Die Anti-HCMV-Wirkung wurde in einem Screening-Testsystem in 96-Well-Mikrotiterplatten unter Zuhilfenahme von humanen embryonalen Lungenfibroblasten (HELF)-Zellkulturen bestimmt. Der Einfluß der Substanzen auf die Ausbreitung des cytopathogenen Effektes wurde im Vergleich zu der Referenzsubstanz Ganciclovir (Cymevene$^R$-Natrium), einem klinisch zugelassenen anti-HCMV-Chemotherapeutikum, bestimmt.

Die in DMSO (Dimethylsulfoxid) gelösten Substanzen (50 mM) werden auf Mikrotiterplatten (96-Well) in Endkonzentrationen von 1000 - 0,00048 µM (micromolar) in Doppelbestimmungen (4 Substanzen/Platte) untersucht. Toxische und cytostatische Substanzwirkungen werden dabei miterfaßt. Nach den entsprechenden Substanzverdünnungen (1:2) auf der Mikrotiterplatte wird eine Suspension von 50 - 100 HCMV-infizierten HELF-Zellen und 3 x $10^4$ nichtinfizierten HELF-Zellen in Eagle's MEM (Minimal Essential Medium) mit 10% fötalem Kälberserum in jedes Näpfchen gegeben, und die Platten bei 37 °C in einem $CO_2$-Brutschrank über 6 Tage inkubiert. Nach dieser Zeit ist der Zellrasen in den substanzfreien Viruskontrollen, ausgehend von 50 - 100 infektiösen Zentren, durch den cytopathogenen Effekt (CPE) des HCMV völlig zerstört (100% CPE). Nach einer Anfärbung mit Neutralrot und Fixierung mit Formalin / Methanol werden die Platten mit Hilfe eines Projektions-Mikroskopes (Plaque-Viewer) ausgewertet. Die Ergebnisse

sind für einige Verbindungen in der folgenden Tabelle zusammengefaßt:

Tabelle: Anti-HCMV (Stamm Davis)-Aktivität und antizelluläre Wirkung

| Bsp.-Nr. | $IC_{50}$ (µM) [1] HCMV | $CIC_{50}$ (µM)[2] HELF | SI [3] |
|---|---|---|---|
| 4 | 1,95 | 62,5 | 32 |
| 19 | 0,45 | 15,6 | 35 |
| 43 | 0,45 | 62,5 | 140 |
| 46 | 7,8 | 500 | 64 |
| 54 | 0,06 | 4 | 67 |
| 69 | 0,06 | 15,6 | 260 |
| 56 | 0,24 | 31 | 130 |
| 75 | 0,015 | 3,9 | 260 |
| Cymevene[R] -Na | 2-4 | 125 | 32 -64 |

1) $IC_{50}$ = Konzentration der erfindungsgemäßen Verbindung, die eine 50%ige Hemmung des CPE bewirkt.

2) $CIC_{50}$ = Höchste Konzentration, die keine offensichtliche antizelluläre Wirkung zeigt.

3) $SI = \frac{CIC_{50}}{IC_{50}}$ = Selektivitätsindex

Es wurde nun gefunden, daß die erfindungsgemäßen Verbindungen, die Vermehrung des HCMV in HELF-Zellen in z.T. 10-50fach niedrigeren Konzentrationen als Cymevene[R]-Natrium hemmen und einen mehrfach höheren Selektivitätsindex aufweisen.

Die erfindungsgemäßen Verbindungen stellen somit wertvolle Wirkstoffe zur Behandlung und Prophylaxe von Erkrankungen durch das humane Cytomegalievirus-Infektionen dar. Als Indikationsgebiete können beispielsweise genannt werden:

1) Behandlung und Prophylaxe von Cytomegalievirus-Infektionen bei Knochenmark- und Organtransplantationspatienten, die an einer HCMV-Pneumonitis, -Enzephalitis, sowie an gastrointestinalen und systemischen HCMV-Infektionen oft lebensbedrohlich erkranken.

2) Behandlung und Prophylaxe von HCMV-Infektionen bei AIDS-Patienten (Retinitis, Pneumonitis, gastrointestinale Infektionen).

3) Behandlung und Prophylaxe von HCMV-Infektionen bei Schwangeren, Neugeborenen und Kleinkindern.

Darüberhinaus wurde überraschend gefunden, daß die Verbindungen der allgemeinen Formel (I) eine Wirkung gegen Retroviren besitzen. Dies wird mit einem HIV-spezifischen Protease-Enzymtest belegt.
Die Ergebnisse der unten aufgeführten Beispiele wurden nach dem in den folgenden Literaturangaben [vgl. Hansen, J., Billich, S., Schulze, T., Sukrow, S. and Mölling, K. (1988), EMBO Journal, Vol, 7, No. 6, pp. 1785 - 1791] beschriebenen HIV-Testsystem ermittelt: Gereinigte HIV-Protease wurde mit synthetischem Peptid, das eine Schnittstelle im Gag-Precursor-Protein imitiert und eine in vivo-Spaltstelle der HIV-Protease darstellt, inkubiert. Die entstandenen Spaltprodukte des synthetischen Peptids wurden über Reverse Phase High Performance Liquid Chromatography (RP-HPLC) analysiert. Die angegebenen $IC_{50}$-Werte beziehen

sich auf die Substanzkonzentration, die unter den oben aufgeführten Testbedingungen eine 50%ige Hemmung der Protease-Aktivität bewirkt.

Tabelle

| $IC_{50}$ (RP-HPLC) (μM) | |
|---|---|
| Bsp.-Nr. | HIV-1 |
| VIII | 19 |
| IX | 160 |
| 17 | 1,6 |
| 20 | 0,013 |
| 42 | 0,15 |
| 49 | 0,26 |
| 50 | 0,14 |
| 51 | 2,1 |
| 52 | 12,0 |
| 55 | 0,12 |
| 56 | 0,24 |
| 57 | 1,5 |
| 58 | 0,0018 |
| 82 | 14 |
| 83 | 0,0024 |

Der neue Wirkstoff kann in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht-toxischer, pharmazeutisch geeigneter Trägerstoffe oder Lösemittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenne Dosierungsspielraum zu erreichen.

Die Formulierungen werden beispielsweise hergestellt durch Verstrecken der Wirkstoffe mit Lösemitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösemittel als Hilfslösemittel verwendet werden können.

Die Applikation erfolgt in üblicher Weise, vorzugsweise oral, parenteral oder topisch, insbesondere perlingual oder intravenös.

Für den Fall der parenteralen Anwendung können Lösungen des Wirkstoffs unter Verwendung geeigneter flüssiger Trägermaterialien eingesetzt werden.

Im allgemeinen hat es sich als vorteilhaft erwiesen, bei intravenöser Applikation Mengen von etwa 0,001 bis 10 mg/kg, vorzugsweise etwa 0,01 bis 5 mg/kg Körpergewicht zur Erzielung wirksamer Ergebnisse zu verabreichen, und bei oraler Applikation beträgt die Dosierung etwa 0,01 bis 25 mg/kg, vorzugsweise 0,1 bis 10 mg/kg Köpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannnte obere Grenze überschrittten werden muß. Im Falle der Applikation größerer Mengen kann es empfehlenswert sein, diese in mehreren Einzelgaben über den Tag zu verteilen.

Als Enzyminhibitoren sind die erfindungsgemäßen Verbindungen einsetzbar in allen Bereichen die allgemein für Inhibitoren bekannt sind. Gemeint dabei ist zum Beispiel der Einsatz als Affinitätslabel für die Affinitätschromomatographie bei der Reinigung von Proteasen. Sie können aber auch als Hilfsmittel dienen für die Aufklärung von Reaktionsmechanismen sowie zur Verbesserung der Spezifität von diagnostischen Verfahren.

Anhang zum experimentellen Teil

I. Aminosäuren

Im allgemeinen erfolgt die Bezeichnung der Konfiguration durch das Vorausstellen eines L bzw. D vor der Aminosäureabkürzung, im Fall des Racemats durch ein D,L-, wobei zur Vereinfachung bei L-Aminosäuren die Konfigurationsbezeichnung unterbleiben kann und dann nur im Fall der D-Form bzw. des D,L-Gemisches eine explizierte Bezeichnung erfolgt.

| | |
|---|---|
| Arg | L-Arginin |
| Ile | L-Isoleucin |
| Leu | L-Leucin |
| Phe | L-Phenylalanin |
| Val | L-Valin |

II. Abkürzungen

| | |
|---|---|
| Z | Benzyloxycarbonyl |
| Boc | tert.Butyloxycarbonyl |
| CMCT | 1-Cyclohexyl-3-(2-morpholino-ethyl)-carbodiimidmetho-p-toluolsulfonat |
| DCC | Dicyclohexylcarbodiimid |
| DMF | Dimethylformamid |
| HOBT | 1-Hydroxybenzotriazol |
| Ph | Phenyl |
| THF | Tetrahydrofuran |
| DMSO | Dimethylsulfoxid |
| Fmoc | 9-Fluorenylmethoxycarbonyl |

III. Liste der verwendeten Laufmittelgemische zur Chromatographie

| | |
|---|---|
| I: | Dichlormethan:Methanol |
| II: | Toluol:Ethylacetat |
| III: | Acetonitril:Wasser |

Ausgangsverbindungen

Beispiel I

(2S)-2-Amino-3-phenyl-propan-1-ol Hydrochlorid

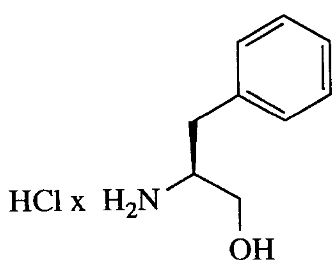

Eine Lösung von 20,10 g (80,00 mmol) (S)-2-(tert.Butoxycarbonylamino-1-phenyl-propan-1-ol [J. Med. Chem. 33, 2707 (1990)] in 200 ml einer 4 N Lösung gasförmigen Chlorwasserstoffs in wasserfreiem Dioxan wird 30 min bei Raumtemperatur gerührt. Danach gibt man 60 ml Toloul zu und engt im Vakuum ein. Dieser Vorgang wird noch zweimal wiederholt, dann wird der Rückstand mit wenig Ether verrieben, abgesaugt und im Hochvakuum über KOH getrocknet. Man erhält 14,14 g (94% der Theorie) der Titelverbindung als farblose Kristalle.
Schmp.: 148-150 °C (Ether)
$R_f$ = 0,25 (Acetonitril : Wasser 9:1)

MS (DCI, $NH_3$) m/z = 152 $(M+H)^+$
IR (KBr) 3357, 2928, 1571, 1495, 1456, 1026, 738, 708 $cm^{-1}$
$[\alpha]^{20}_D$ = -4,2° (c = 2,94, $CH_3OH$)
$^1$H-NMR (300 MHz, $CD_3OD$) $\delta$ = 2,95 (d, 2H, J = 7,5 Hz, $CH_2$); 3,50 (m, 2H); 3,70 (m, 1H); 7,30 (m, 5H, Ph).
$C_9H_{13}NO$ x HCl (187,67)

Beispiel II

(2S)-2-[N-(tert.Butoxycarbonyl)-5-valinyl]amino-3-phenyl-propan-1-ol

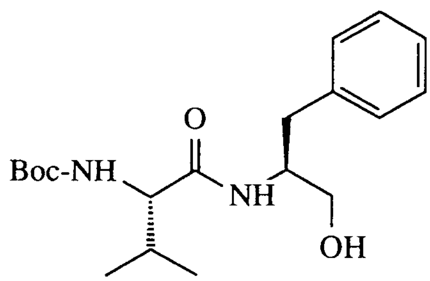

Eine auf 0°C gekühlte, gerührte Lösung von 18,01 g (82,90 mmol) N-(tert.Butoxycarbonyl)-L-valin und 12,69 g (82,90 mmol) HOBT in 300 ml wasserfreiem Dichlormethan wird mit 16,32 g (79,10 mmol) DCC versetzt und 5 min gerührt. Danach wird eine Lösung von 14,14 g (75,40 mmol) der Verbindung aus Beispiel I und 20.73 g (188,50 mmol) N-Methylmorpholin in 300 ml Dichlormethan zugetropft. Das Kühlbad wird entfernt und die Reaktionsmischung darf 2 h bei Raumtemperatur gerührt werden. Das Ende der Reaktion wird dünnschichtchromatographisch festgestellt. Man trennt den entstandenen Harnstoff durch Filtration ab, engt das Filtrat im Vakuum ein und reinigt das Rohprodukt durch Chromatographie an 450 g Kieselgel (Dichlormethan: Methanol 95:5). Man erhält 25,15 g (95% der Theorie) der Titelverbindung als farblose Kristalle.
Schmp.: 143°C
$R_f$ = 0,29 (Dichlormethan : Methanol 95:5)
MS (FAB) m/z = 351 $(M+H)^+$
IR (KBr) 3340, 2933, 1686, 1657, 1523, 1368, 1311, 1246, 1172, 1044, 698 $cm^{-1}$
$[\alpha]^{20}_D$ = -42,1° (c = 0,401, $CH_3OH$)
$^1$H-NMR (300 MHz, $CD_3OD$) $\delta$ = 0,87 (t, J = 7 Hz, 6H $[\underline{CH_3}]_2CH$); 1,44 (s, 9H, $CH_3$-C); 1,93 (m, 1H, $[CH_3]_2\underline{CH}$); 2,74 (dd, J = 8, 14 Hz, 1H, $\underline{CH_2}Ph$); 3,92 (dd, J = 6 Hz, 14 Hz, 1H $\underline{CH_2}Ph$); 3,50 (d, J = 6 Hz, 2H, $\underline{CH_2}OH$); 3,79 (d, J = 7 Hz, 1H, NCHCO); 4,12 (m, 1H, NCH); 7,23 (m, 5H, Ph).
$C_{19}H_{30}N_2O_4$ (350,47)

Beispiel III

(2S)-2-(N-S-Valinyl)amino-3-phenyl-propan-1-ol

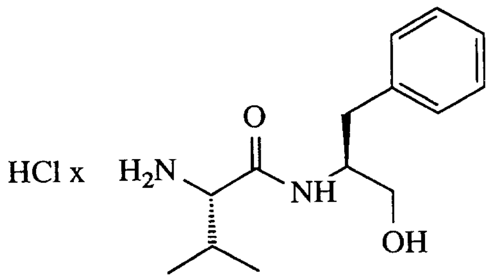

Eine Lösung von 25,15 g (75,6 mmol) der Verbindung aus Beispiel I in 180 ml wasserfreien Dioxan wird mit 180 ml einer 4 N Lösung gasförmigen Chlorwasserstoffs in wasserfreiem Dioxan versetzt und 30 min bei Raumtemperatur gerührt. Danach gibt man 150 ml Toluol zu und engt im Vakuum ein. Dieser Vorgang wird noch zweimal wiederholt, dann wird der Rückstand mit 300 ml Ether verrieben, abgesaugt und im Hochvakuum über KOH getrocknet. Man erhält 20,12 g (98% der Theorie) der Titelverbindung als farblose Kristalle.
Schmp.: ab 100 ° C (Zers.)
$R_f$ = 0,19 (Acetonitril : Wasser 9:1)
MS (DCI, $NH_3$) m/z = 251 $(M+H)^+$
IR (KBr) 3267, 2931, 1670, 1571, 1496, 1259, 1120, 1040, 870 $cm^{-1}$
$[\alpha]^{20}_D$ = 2,5 ° (c = 0,375, $CH_3OH$)
$^1$H-NMR (300 MHz, $CDCl_3$) $\delta$ = 1,03, 1,07 (d, 7Hz, 6H, $[\underline{CH_3}]_2CH$); 2,20 (m, 1H, $[CH_3]_2\underline{CH}$); 2,88 (AB, J = 7,5, 15 Hz, 2H, $\underline{CH_2}Ph$); 3,54 (m, 2H, $\underline{CH_2}OH$); 3,63 (d, J = 6,5 Hz, 1H, NCHCO); 4,16(1H, NCH); 7,28 (m, 5H, Ph).
$C_{14}H_{22}N_2O_2$ x HCl (286,80)

| | | | |
|---|---|---|---|
| Ber.: | C 58,63 | H 8,08 | N 9,77 |
| Gef.: | C 58,7 | H 8,3 | N 9,5 |

Beispiel IV

(2S)-2-[Nα-(tert.Butoxycarbonyl)-$N^G$-(4-methyl-phenylsulfonyl)-S-arginyl-S-valinyl]amino-3-phenyl-propan-1-ol

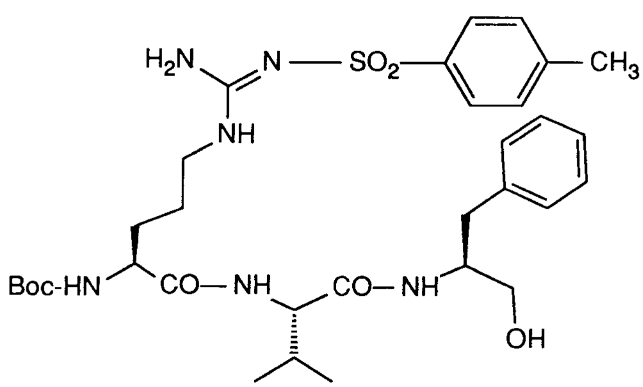

Eine auf 0 ° C gekühlte, gerührte Lösung von 18,64 g (43,51 mmol) $N_\alpha$-(tert.Butoxycarbonyl)-$N^G$-(4-methylphenylsulfonyl)-S-arginin und 6,66 g (43,50 mmol) HOBT in 190 ml wasserfreiem Dichlormethan und 19 ml

DMF wird mit 8,57 g (41,50 mmol) DCC versetzt und 5 min gerührt. Danach wird eine Lösung von 11,33 g (39,50 mmol) der Verbindung aus Bespiel III und 17,38 ml (158,10 mmol) N-Methylmorpholin in 113 ml Dichlormethan und 11 ml DMF zugetropft. Das Kühlbad wird entfernt und die Reaktionsmischung darf 1 h bei Raumtemperatur gerührt werden. Das Ende der Reaktion wird dünnschichtchromatographisch festgestellt. Man trennt den entstandenen Harnstoff durch Filtration ab, engt das Filtrat im Vakuum ein und reinigt das Rohprodukt durch Chromatographie an 500 g Kieselgel (Dichlormethan : Methanol 9:1). Man erhält 18,98 g (73% der Theorie) der Titelverbindung als farblosen Schaum.
$R_F$ = 0,35 (Dichlormethan: Methanol 9:1)
MS (FAB) m/z = 661 $(M+H)^+$
IR (KBr) 3336, 2967, 1654, 1544, 1253, 1168, 1131, 1082, 676 $cm^{-1}$
$[\alpha]^{20}_D$ = -32,7° (c = 0,895, $CH_3OH$)
$^1$H-NMR (250 MHz, $CD_3OD$) $\delta$ = 0,89 (m, 6H, $[\underline{CH_3}]_2CH$); 1,43 (s, 9H, $CH_3$-C); 1,4 - 1,6 (m, 4H, $CH_2$); 1,99 (m, 1H, $[CH_3]_2\underline{CH}$); 2,38 (s, 3H, $CH_3$); 2,70 (dd, J = 10, 16 Hz, 1H, $\underline{CH_2}Ph$); 2,90 (dd, J = 7,5, 15 Hz, 1H, $CH_2Ph$); 3,13 (m, 2H, $CH_2N$); 3,50 (d, J = 7 Hz, 2H, $CH_2O$); 3,72 (m, 1H, NCHCO); 4,0 - 4,2 (m, 2H, NCHCO, NCH); 7,20 (m, 5H, Ph); 7,30, 7,73 (AB, J = 10 Hz, 4H, H arom.)
$C_{32}H_{48}N_6O_7S$ (660,85)

| | | | |
|---|---|---|---|
| Ber.: | C 58,16 | H 7,32 | N 12,72 |
| Gef.: | C 58,3 | H 7,4 | N 12,6 |

Beispiel V

(2S)-2-[$N^G$-(4-Methyl-phenylsulfonyl)-S-arginyl-S-valinyl]amino-3-phenylpropan-1-ol Dihydrochlorid

2 HCl x 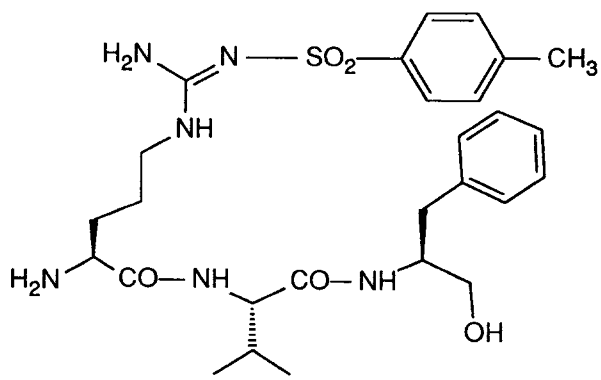

Wie für Beispiel I beschrieben erhält man aus 18,90 g (28,60 mmol) der Verbindung aus Beispiel IV 18,68 g (99% der Theorie) der Titelverbindung als farbloses Pulver. Schmp.: 161-162°C
$R_f$ = 0,36 (Acetonitril : Wasser 9:1)
MS (FAB) m/z = 561 $(M+H)^+$
IR (KBr) 2964, 1655, 1560, 1342, 1171, 1090, 1041, 666 $cm^{-1}$
$[\alpha]^{20}_D$ = 2,3° (c = 0,983, $CH_3OH$)
$^1$H-NMR (250 MHz, $DC_3OD$): $\delta$ = 0,96 (m, 6H, $[\underline{CH_3}]_2CH$); 1,50 (m, 2H, $CH_2$); 1,80 (m, 2H, $CH_2$); 2,03 (m, 1H $[CH_3]_2\underline{CH}$); 2,42 (s, 3H, $CH_3$); 2,68 (dd, J = 8 Hz, 14 Hz, 1H, $\underline{CH_2}Ph$); 2,86 (dd, J = 6 Hz, 14 Hz, 1H, $CH_2Ph$); 3,12(t, J = 6,5 Hz, 2H, $CH_2N$); 3,51 (d, J = 6 Hz, 2H, $\underline{CH_2O}$); 3,97 (m, 1H, NCHCO-Arg); 4,07 (m, 1H, NCH); 4,18 (d, J = 7,5 Hz, NCHCO-Val); 7,18 (m, 5H, Ph); 7,45, 7,87 (AB, J = 10 Hz, 4 H, H arom.)
$C_{27}H_{40}N_6O_5S$ x 2 HCl (633,66)

| | | | |
|---|---|---|---|
| Ber.: | C 51,18 | H 6,68 | N 13,26 |
| Gef.: | C 49,9 | H 6,8 | N 13,3 |

Beispiel VI

Nα-(tert.Butoxycarbonyl)-$N^G$-(4-methyl-phenylsulfonyl)-S-arginyl-S-valinyl-S-phenylalanin-methylester

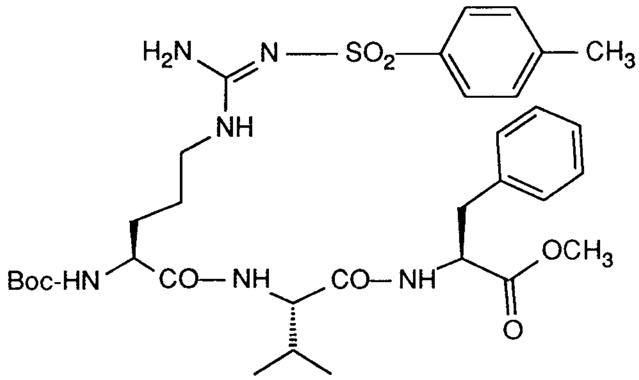

Wie für Beispiel IV beschrieben erhält man aus 14,14 g (33,00 mmol) $N_\alpha$-(tert.Butoxycarbonyl)-$N^G$-(4-methyl-phenylsulfonyl)-S-arginin und 9,02 g (28,70 mmol) S-Valinyl-S-phenylalanin-methylester Hydrochlorid [EP 77 029; A. Orlowska et al. Pol. J. Chem. 54, 2329 (1980)] nach 3 h bei Raumtemperatur 13,66 g (69% der Theorie) der Titelverbindung als farblosen Schaum.
$R_f$ = 0,33 (Ethylacetat)
MS (FAB) m/z = 689 $(M+H)^+$
IR (KBr) 3343, 2967, 1740, 1655, 1546, 1254, 1169, 1132, 1083, 676 $cm^{-1}$
$[\alpha]^{20}_D$ = -9,1° (c = 0,389, DMSO)
$^1$H-NMR (250 MHz, $DMSO_{d6}/D_2O$): δ = 0,82 (m, 6H, $[CH_3]_2CH$); 1,49 (s, $CH_3$-C); 1,3 - 1,5 (m, $CH_2$) zusammen 13H, 1,95 (m, 1H, $[CH_3]_2CH$); 2,35 (s, 3H, $CH_3$); 3,05 (m, 4H, $CH_2Ph$, $CH_2N$); 3,60 (s, 3H, $COOCH_3$); 3,89 (m, 1H, NCHCO); 4,49 (m, 1H, NCHCO); 7,15 - 7,30 (m, 5H, Ph); 7,33, 7,68 (AB, J = 10 Hz, 4H, H arom.)

Beispiel VII

$N^G$-(4-Methyl-phenylsulfonyl)-S-arginyl-S-valinyl-S-phenylalanin-methylester Dihydrochlorid

2 HCl x 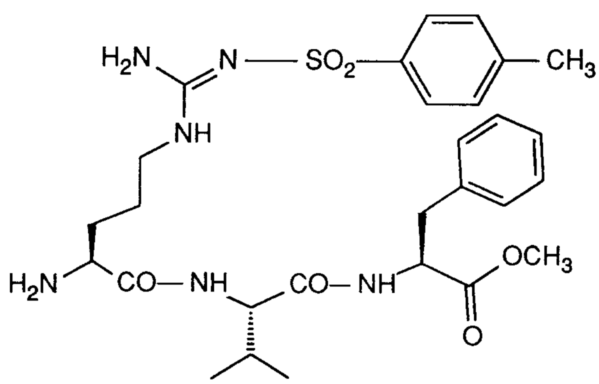

Wie für Beispiel I beschrieben erhält man aus 11,50 g (16,70 mmol) der Verbindung aus Beispiel VI 10,18 g (92% der Theorie) der Titelverbindung als farbloses Pulver. Schmp.: ab 190 °C (Zers.)
$R_f$ = 0,18 (Dichlormethan: Methanol 9:1)
MS (FAB): m/z = 589 $(M+H)^+$
IR (KBr) 2963, 1744, 1670, 1549, 1364, 1218, 1171, 1086, 668 $cm^{-1}$
$[\alpha]^{20}_D$ = 7,6° (c = 0,493, DMSO)
$^1$H-NMR (250 MHz, $DMSO_{d6}/CD_3OD$) δ = 0,97 (d, J = 8 Hz, 6H, $[CH_3]_2CH$); 1,41, 1,62 (m, 4H, $CH_2$); 2,00

(m, 1H, $[CH_3]_2\underline{CH}$); 2,35 (s, 3H, $CH_3$); 2,90 - 3,15 (m, 4H, $CH_2Ph$, $CH_2N$); 3,58 (s, 3H, $COOCH_3$); 3,86 (m, 1H, NHCHCO); 4,25 (m, NCHCO, unter HDO); 4,52 (m, 1H, NCHCO); 7,22 (m, 5H, Ph); 7,30, 7,68 (AB, J = 9 Hz, 4H, H arom.).

Beispiel VIII und Beispiel IX

(2S)-2-{$N_\alpha$-[((1S)-1-Carboxy-2-phenyl-ethyl)aminocarbonyl]-S-arginyl-S-valinyl}amino-3-phenyl-propan-1-al (= α-MAPI)

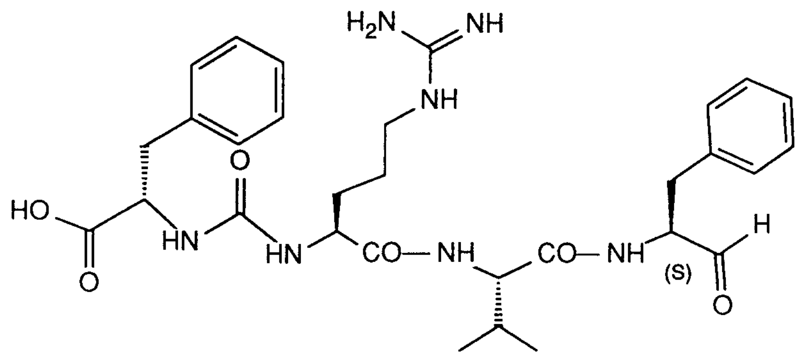

(VIII)

und (2R)-2-{$N_\alpha$-[((1S)-1-Carboxy-2-phenyl-ethyl)aminocarbonyl]-S-arginyl-S-valinyl}amino-3-phenyl-propan-1-al (= β-MAPI)

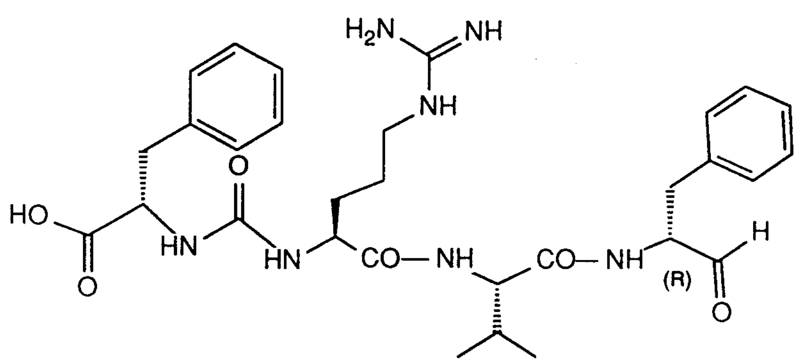

(IX)

Eine Lösung von 3,00 g (5,02 mmol) der Verbindung aus Beispiel 3 in 45 ml DMSO und 6,3 ml (45,20 mmol) Triethylamin wird mit 3,59 g (22,6 mmol) Pyridin-Schwefeltrioxid-Komplex versetzt und 1 h bei Raumtemperatur gerührt.

Danach wird das Reaktionsgemisch in 400 ml Ether eingerührt, wobei sich ein Öl abscheidet. Die Etherphase wird abdekandiert und das zurückbleibende Öl wird an 500 g Kieselgel (Chloroform : Methanol : Wasser : Essigsäure = 13:3:1:1) chromatographiert. Die produkthaltigen Fraktionen werden gesammelt, mit 100 ml Toluol versetzt und im Vakuum eingeengt. Der Rückstand wird in 50 ml Toluol aufgenommen, und das Toluol wird im Vakuum abgedampft. Der Rückstand wird mit 80 ml wasserfreiem Acetonitril verrieben, wobei ein kristalliner Niederschlag entsteht. Dieser wird durch Filtration abgetrennt und 10 ml Acetonitril gewaschen und mit 2 x 10 ml Ether verrieben, abgesaugt und im Hochvakuum getrocknet. Man erhält 850 mg (28%) der Titelverbindung als Gemisch der diastereomeren Aldehyde (α, β-MAPI).
Schmp.: 214 °C (Zers.)
$R_F$ = 0,29 (Chloroform: Methanol : Wasser : Essigsäure 13:3:1:1).
Zur Trennung der diastereomeren Aldehyde löst man 150 mg des Gemisches in 1 ml Ameisensäure und chromatographiert an einer HPLC-Anlage (Säule: Dynamax$^R$-60 A, 21,4 x 250 mm, Rainin Instrument Company Nr. 83-221-C; Typ: C18, Partikelgröße: 8 μm; Porengröße: 60 A, Fluß: 10 ml/min; Eluent: Wasser: Methanol 1:1 + 0,05% Trifluoressigsäure). Die produkthaltigen Fraktionen werden gesammelt und gefriergetrocknet. Man erhält 56 mg des (2S)-Isomers (α-MAPI) (VIII)
MS (FAB) m/z = 596 $(M+H)^+$, 749 $(M+NBA+H)^+$

IR (KBr): 3380, 2990, 2930, 1640, 1546, 1203, 700 $cm^{-1}$
37 mg einer Mischfraktion (α,β-MAPI) (VIII + IX)und 39 mg des (2R)-Isomers (β-MAPI) (IX)
MS (FAB) m/z = 596 $(M+H)^+$, 749 $(M+NBA+H)^+$
IR (KBr) 3407, 2998, 2935, 1654, 1560, 1204, 701 $cm^{-1}$.
Die $^1$H-NMR spektroskopischen Daten sind identisch mit den aus Streptomyces nigrescens WT-27 erhaltenen diastereomeren Aldehyden:
(4) α-MAPI: (T. Watanabe, K. Fukuhara, S. Murao, Tetrahedron Lett. 625 (1979)
(5) β-MAPI: (T. Watanabe, K. Fukuhara, S. Murao, Tetrahedron 38, 1775 (1982)

Beispiel X

$N_\alpha$-[((1S)-1-Carboxy-2-phenyl-ethyl)aminocarbonyl]-S-arginyl-S-valinyl-S-phenylalanin

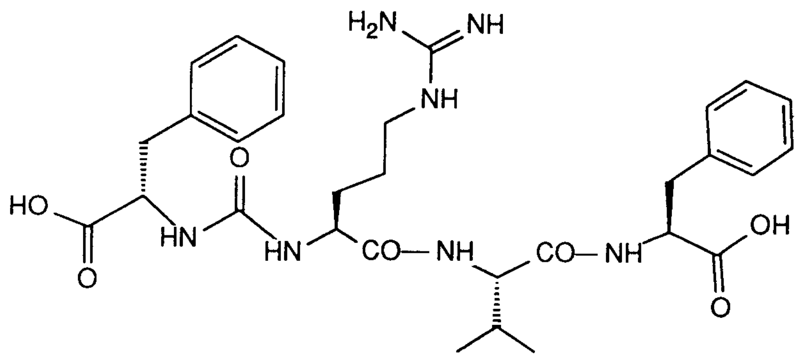

Wie für Beispiel 2 beschrieben erhält man aus 330 mg (0,52 mmol) der Verbindung aus Beispiel 8 und 96 mg (2,26 mmol) Lithiumhydroxid-Hydrat in einem Gemisch aus 2 ml THF und 2,2 ml Wasser, 221 mg (70%) der Titelverbindung als farbloses Pulver.
Schmp.: ab 217 °C (Zers.)
$R_f$ = 0,29 (Chloroform: Methanol 7:3)
MS (FAB) m/z = 612 $(M+H)^+$
IR (KBr) 3310, 1639, 1543, 1390, 1219, 701 $cm^{-1}$
$[\alpha]^{20}_D$ = 31,6 ° (c = 0,0558, DMSO)
$^1$H-NMR (250 MHz, $DMSO_{d6}$ / $D_2O$) δ = 0,72, 0,80 (d, J = 6,5 Hz, 6H, $[CH_3]_2CH$); 1,40 - 1,80 (m, 4H, $CH_2$); 1,92 (m, 1H, $[CH_3]_2CH$); 2,80 - 3,10 (m, 6H, $CH_2Ph$, $CH_2N$); 3,91 (d, J = 6,5 Hz, 1H, NCHCO-Valin); 4,14 (m, 1H, NCHCO); 4,23 (m, 2H, NCHCO); 7,10 - 7,30 (m, 10H, Ph).

Herstellungsbeispiele

Beispiel 1

(2S)-2-{$N_\alpha$-[((1S)-1-Methoxycarbonyl-2-phenyl-ethyl)aminocarbonyl]-$N^G$-(4-methoxy-phenylsulfonly)-S-arginyl-S-valinyl}amino-3-phenyl-propan-1-ol

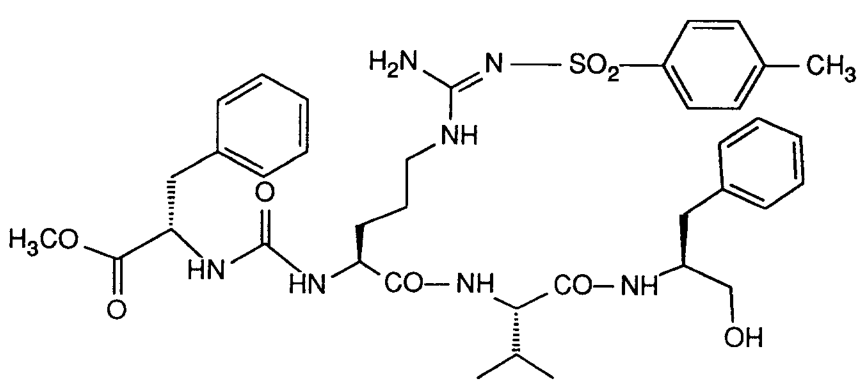

Eine gerührte Suspension von 2,00 g (3,16 mmol) der Verbindung aus Beispiel V in 60 ml wasserfreiem Dichlormethan wird mit 1,01 ml (7,26 mmol) Triethylamin versetzt, wobei eine klare Lösung entsteht. Dazu gibt man 712 mg (3,47 mmol) (2S)-Methyl-2-isocyanato-3-phenyl-propanoat [US 4 929 736; H. Danda et al., Chem. Express 6, 261 (1991)] und rührt 30 min bei Raumtemperatur nach. Man gibt 40 ml Toluol zu, engt die Reaktionsmischung im Vakuum ein und reinigt den Rückstand durch Chromatographie an 220 g Kieselgel (Dichlormethan : Methanol 92:8). Man erhält 2,12 g (88%) der Titelverbindung als farblose Kristalle. Schmp.: 153 °C

$R_f$ = 0,33 (Dichlormethan: Methanol 9:1)

MS (FAB) m/z = 766 $(M+H)^+$

IR (KBr) 3342, 2980, 1742, 1624, 1550, 1269, 1133, 1082, 674 $cm^{-1}$

$[\alpha]^{20}_D$ = -9,0 ° (c = 0,85, DMSO)

$^1$H-NMR (250 MHz, $DMSO_{d6}/D_2O$): δ = 0,78 (m, 6H, $[CH_3]_2CH$); 1,30 - 1,55 (m, 4H, $CH_2$); 1,88 (m, 1H, $[CH_3]_2CH$); 2,35 (s, 3H, $CH_3$); 2,60 (dd, J = 9 Hz, 14 Hz, 1H, $CH_2Ph$); 2,86 (dd, J = 6 Hz, 14 Hz, 1H, $CH_2Ph$); 2,92 - 3,10 (m, 4H, $CH_2Ph$, $CH_2N$); 3,32 (m, 2H, $CH_2O$); 3,59 (s, 3H, $COOCH_3$); 3,90 - 4,10 (m, 3H, NCHCO, NCH); 4,40 (m, 1H, NCHCO); 7,13 - 7,33 (m, 12H, H arom.); 7,65 (d, J = 10 Hz, H arom.).

$C_{38}H_{51}N_7O_8S$ (765,95)

| | | | |
|---|---|---|---|
| Ber.: | C 59,59 | H 6,71 | N 12,80 |
| Gef.: | C 59,3 | H 6,7 | N 12,8 |

Beispiel 2

(2S)-2-{$N_\alpha$-[((1S)-1-Carboxy-2-phenyl-ethyl)aminocarbonyl]-$N^G$-(4-methyl-phenylsulfonyl)-S-arginyl-S-valinyl}amino-3-phenyl-propan-1-ol

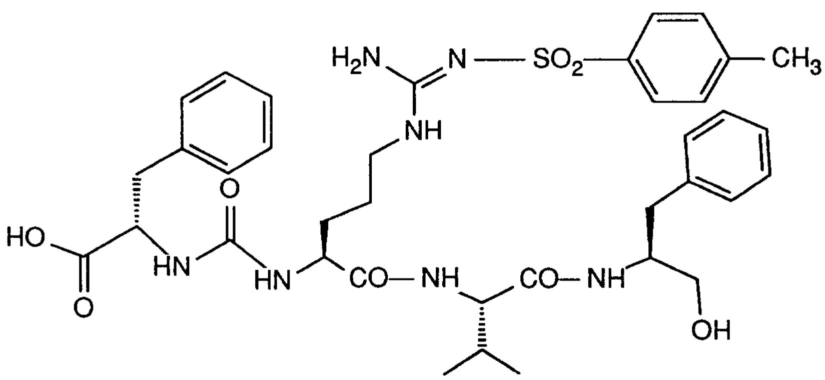

Eine Lösung von 4,96 g (6,48 mmol) der Verbindung aus Beispiel 1 in 16 ml THF und 20 ml Wasser wird mit 544 mg (12,96 mmol) Lithiumhydroxid-hydrat versetzt und 20 min bei Raumtemperatur gerührt. Danach wird die Reaktionsmischung in 100 ml Ethylacetat geschüttet. Die organische Phase wird abgetrennt und die wäßrige Phase erneut mit 30 ml Ethylacetat extrahiert. Die wäßrige Phase wird am Rotationsverdampfer von Lösemittelresten befreit und mit 0,5 H Salzsäre auf pH 5,2 gestellt. Der entstandene Niederschlag wird 10 min gut durchgerührt, durch Filtration abgetrennt und im Hochvakuum zunächst über KOH, dann über Sicapent getrocknet. Man erhält 4,14 g (85%) der Titelverbindung als farbloses Pulver.

Schmp.: ab 140 °C (Zers.)

$R_f$ = 0,21 (Acetonitril: Wasser 9:1)

MS (FAB): m/z = 732 $(M+H)^+$, 774 $(M+Na)^+$

IR (KBr) 3346, 2962, 1730, 1637, 1549, 1253, 1132, 1082, 700 $cm^{-1}$

$[\alpha]^{20}_D$ = -5,5 ° (c = 0,704, DMSO)

$^1$H-NMR (300 MHz, DMSO-$d_6$, $D_2O$): δ = 0,75 (m, 6H, $[CH_3]_2CH$); 1,30 - 1,60 (m, 4H, $CH_2$); 1,90 (m, 1H, $[CH_3]_2CH$); 2,35 (s, 3H, $CH_3$); 2,61 (dd, J = 7 Hz, 13 Hz, 1H, $CH_2Ph$); 2,80 - 3,10 (m, 5H, $CH_2Ph$, $CH_2N$); 3,31 (m, 2H, $CH_2O$); ca. 4,0 unter HDO: (NCHO, NCH); 4,38 (m, 1H, NCHCO); 7,10 - 7,30 (m, 12H, H

arom.); 7,67 (d, J = 10 Hz, 2H, H arom.)
$C_{37}H_{49}N_7O_8S$ (751,91)

| | | | |
|---|---|---|---|
| Ber.: | C 59,10 | H 6,57 | N 13,04 |
| Gef.: | C 60,1 | H 6,7 | N 12,8 |

Beispiel 3

(2S)-2-{$N_\alpha$-[((1S)-1-Carboxy-2-phenyl-ethyl)aminocarbonyl]-S-arginyl-S-valinyl}amino-3-phenyl-propan-1-ol Hydrofluorid

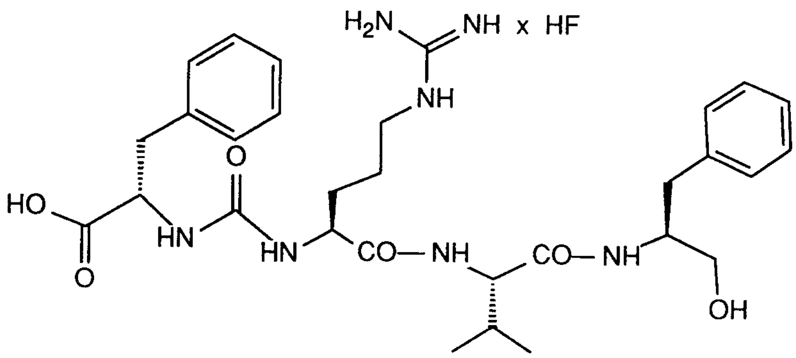

Eine Lösung von 2,00g (2,66 mmol) der Verbindung aus Beispiel 2 und 1,00 g (9,25 mmol) p-Kresol in 20 ml wasserfreier Flußsäure wird 20 min bei 0 °C gerührt. Danach wird die Flußsäure im Vakuum abgezogen und der Rückstand wird in 60 ml Essigsäure gelöst und in ein Gemisch aus 200 ml Ethylacetat und 600 ml Wasser eingerührt. Die Wasserphase wird abgetrennt und gefriergetrocknet. Man erhält 1,48 g (94%) der Titelverbindung als farbloses Lyophilisat.
$R_f$ = 0,27 (Acetonitril : Wasser 4:1)
MS (FAB) m/z = 598 $(M+H)^+$
IR (KBr) 3380, 1655, 1637, 1560, 1204, 700 $cm^{-1}$
$[\alpha]^{20}_D$ = -4,9 ° (c = 0,635, DMSO)
$^1$H-NMR (250 MHz, DMSO-$d_6$, $D_2O$): $\delta$ = 0,78 (s, 6H, $[\underline{CH_3}]_2CH$); 1,45 - 1,65 (m, 4H, $CH_2$); 1,90 (m, 1H, $[CH_3]_2\underline{CH}$); 2,61 (dd, 9,4 Hz, 15 Hz, 1H, $CH_2Ph$); 2,80 - 3,10 (m, 5H, $CH_2Ph$, $CH_2N$); 3,85 (m, 2H, $CH_2O$); 3,95 - 4,15 unter HDO (NCHCO, NCH); 4,48 (m, 1H, NCHCO); 7,15 - 7,35 (m, 10H, H arom.).

Beispiel 4 und Beispiel 5

(2R)-2-{$N_\alpha$-[(1S)-1-Methoxycarbonyl-2-phenyl-ethyl)aminocarbonyl]-$N^G$-(4-methyl-phenylsulfonyl)-S-arginyl-S-valinyl}amino-3-phenyl-propan-1-al

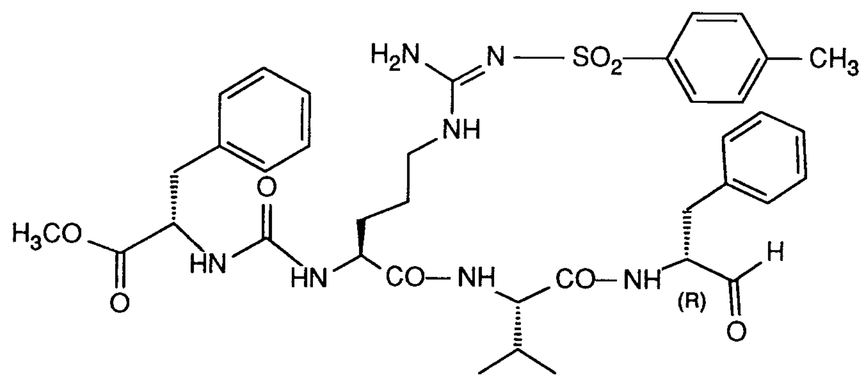

(4)

und (2S)-2-{$N_\alpha$-[((1S)-1-Methoxycarbonyl-2-phenyl-ethyl)aminocarbonyl]-$N^G$-(4-methyl-phenylsulfonyl)-S-arginyl-S-valinyl}amino-3-phenyl-propan-1-al

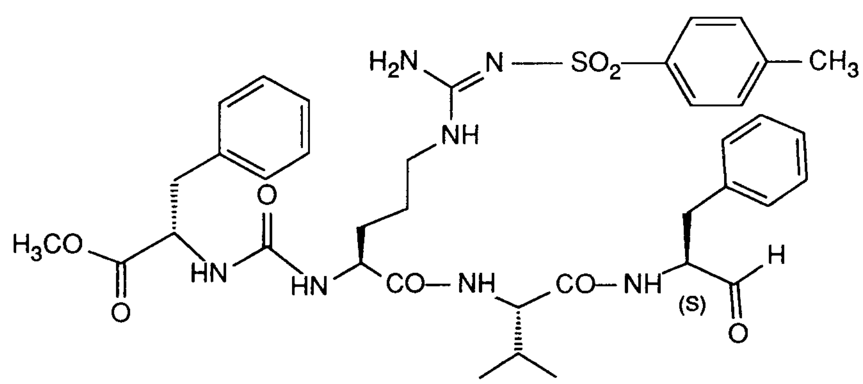

(5)

Eine Lösung von 500 mg (0,65 mmol) der Verbindung aus Beispiel 1 in 4,8 ml DMSO und 0,82 ml (5,87 mmol) Triethylamin wird mit 468 mg (2,94 mmol) Pyridin-Schwefeltrioxid-Komplex versetzt und 30 min bei Raumtemperatur gerührt. Danach wird das Reaktionsgemisch in 50 ml Ether eingerührt. Das Gemisch darf kurze Zeit stehen, wobei sich ein Öl abscheidet. Die Etherphase wird abdekantiert, und das Öl wird in 20 ml Toluol aufgenommen. Das Toluol wird im Vakuum eingedampft, und der Rückstand wird an 250 g Kieselgel (Dichlormethan: Methanol 9:1) chromatographiert.
Man erhält 179 mg (36%) des unpolaren Diastereomers (4) als farblose Kristalle.
Schmp.: 126 °C (Zers.)
$R_f$ = 0,39 (Dichlormethan: Methanol 9:1)
MS (FAB) m/z = 764 $(M+H)^+$
IR (KBr) 3346, 2959, 1736, 1637, 1550, 1256, 1132, 1082, 700 $cm^{-1}$
$[\alpha]^{20}_D$ = 26,4 ° (c = 0,489, DMSO)
$^1$H-NMR (250 MHz, DMSO-$d_6$): $\delta$ = 0,65 (t, J = 7 Hz, 6H, $[CH_3]_2CH$); 1,3 - 1,6 (m, 4H, $CH_2$); 1,80 (m, 1H, $[CH_3]_2CH$); 2,32 (s, 3H, $CH_3$); 2,65 - 3,10 (m, 6H, $CH_2Ph$, $CH_2N$); 3,58 (s, 3H, $COOCH_3$); 4,12 (m, 2H, NCHCO); 4,38 (m, 2H, NCHCO); 6,43 (d, J = 9 Hz, 1H, NH); 7,10 - 7,30 (m, 12H, H arom.); 7,62 (d, J = 8 Hz, 2H, H arom.); 7,28 (d, J = 9 Hz, 1H, NH); 8,46 (d, J = 7,5 Hz, 1H, NH); 9,48 (s, 1H, CHO).

Außerdem erhält man 168 mg (30%) des polaren Diastereomers (5) als farblose Kristalle.
Schmp.: 156 °C (Zers.)
$R_f$ = 0,35 (Dichlormethan : Methanol 9:1)
MS (FAB) m/z = 764 $(M+H)^+$
IR (KBr) 3357, 2964, 1736, 1625, 1550, 1275, 1133, 1082, 672 $cm^{-1}$
$[\alpha]^{20}_D$ = -7,9° (c = 0,538, DMSO)
$^1H$-NMR (250 MHz, DMSO-$d_6$): δ = 0,78 (m, 6H, $[CH_3]_2CH$); 1,20 - 1,60 (m, 4H, $CH_2$); 1,89 (m, 1H, $[CH_3]_2CH$); 2,32 (s, 3H, $CH_3$); 2,70 - 3,10 (m, 6H, $CH_2Ph$, $CH_2N$); 3,57 (s, 3H, $COOCH_3$); 4,10 (m, 2H, NCHCO); 4,38 (m, 2H, NCHCO); 6,40 (d, J = 8 Hz, 1H, NH); 7,10 - 7,30 (m, 12H, H arom.); 7,62 (d, J = 8 Hz, 2H, H arom.); 7,29 (d, J = 9 Hz, 1H, NH); 8,42 (d, J = 6,5 Hz, 1H, NH); 9,42 (s, 1H, CHO).

Beispiel 6

$N_\alpha$-[((1S)-1-Methoxycarbonyl-2-phenyl-ethyl)aminocarbonyl]-$N^G$-(4-methyl-phenylsulfonyl)-S-arginyl-S-valinyl-S-phenylalanin-methylester

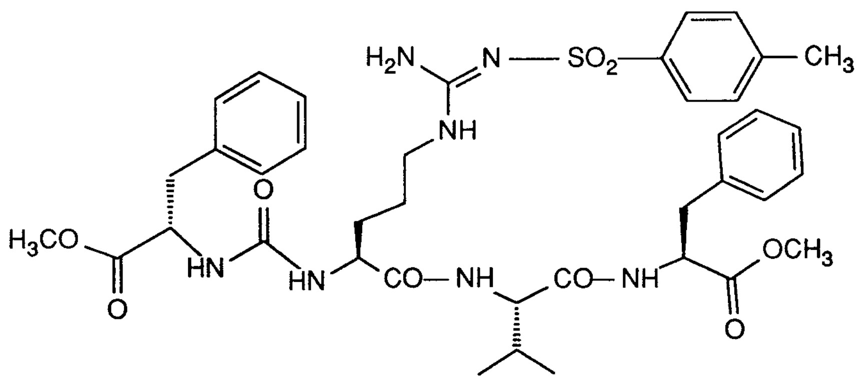

Wie für Beispiel 1 beschrieben, erhält man aus 9,85 g (14,90 mmol) der Verbindung aus Beispiel VII und 3,67 g (17,88 mmol) (2S)-Methyl-2-isocyanato-3-phenyl-propanoat [US 4 929 736] 9,75 g (85%) der Titelverbindung als farblose Kristalle.
Schmp.: 200-201 °C
$R_f$ = 0,44 (Dichlormethan: Methanol 9:1)
MS (FAB) m/z = 794 $(M+H)^+$
IR (KBr) 3316, 2968, 1748, 1626, 1551, 1276, 1213, 1136, 1084, 700, 672 $cm^{-1}$
$[\alpha]^{20}_D$ = 2,0° (c = 0,58, DMSO)
$^1H$-NMR (200 MHz, DMSO-$d_6$) δ = 0,78 (t, J = 5,5 Hz, 6H, $[CH_3]_2CH$); 1,20 - 1,55 (m, 4H, $CH_2$); 2,32 (s, 3H, $CH_3$); 2,80 - 3,10 (m, 4H, $CH_2Ph$, $CH_2N$); 3,55 (s, 3H, $COOCH_3$); 3,59 (s, 3H, $COOCH_3$); 4,18 (m, 2H, NCHCO); 4,31 - 4,52 (m, 2H, NCHCO); 6,41 (d, J = 7,5 Hz, 1H, NH); 7,10 - 7,30 (m, 7H, H arom.); 7,63 (d, J = 9 Hz, 2H, H arom.); 7,78 (d, J = 9,5 Hz, NH); 8,42 (d, J = 7 Hz, NH)
$C_{39}H_{51}N_7O_9S$ (793,95)

| | | | |
|---|---|---|---|
| Ber.: | C 59,00 | H 6,47 | N 12,35 |
| Gef.: | C 58,9 | H 6,5 | N 12,3 |

Beispiel 7

$N_\alpha$-[((1S)-1 Carboxy-2-phenyl-ethyl)aminocarbonyl]-$N^G$-(4-methyl-phenylsulfonyl)-S-arginyl-S-valinyl-S-phenylalanin

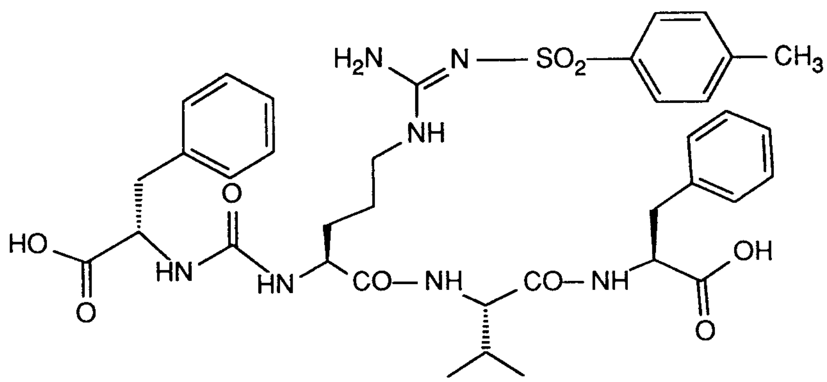

Wie für Beispiel 2 beschrieben erhält man aus 500 mg (0,63 mmol) der Verbindung aus Beispiel 6 und 106 mg (2,52 mmol) Lithiumhydroxid-Hydrat in einem Gemisch aus 3,5 ml THF und 4 ml Wasser 380 mg (78%) der Titelverbindung als farbloses Pulver.
Schmp.: ab 143 °C (Zers.)
$R_f$ = 0,26 (Acetonitril : Wasser 85:15)
MS (FAB) m/z = 766 $(M+H)^+$, 772 $(M+Li)^+$
IR (KBr) 3356, 2965, 1743, 1638, 1550, 1400, 1252, 1131, 1081, 700 $cm^{-1}$
$[\alpha]^{20}_D$ = 10,5 ° (c = 0,633, DMSO)
$^1$H-NMR (250 MHz, $DMSO_{d6}$ / $D_2O$) $\delta$ = 0,78 (m, 6H, $[\underline{CH_3}]_2CH$); 1,15 - 1,60 m (4H, $CH_2$); 1,93 (m, 1H, $[CH_3]_2\underline{CH}$); 2,34 (s, 3H, $CH_3$); 2,80 - 3,10 (m, 6H, $CH_2Ph$, $\underline{CH_2}N$); 4,11 (m, 2H, NCHCO); 4,30 - 4,45 (m, 2H, NCHCO); 7,20 (m, 12H, H arom.); 7,65 (d, J = 8 Hz, 2H, H arom.)

Beispiel 8

$N_\alpha$-[((1S)-1-Methoxycarbonyl-2-phenyl-ethyl)aminocarbonyl]-S-arginyl-S-valinyl-S-phenylalanin-methylester Hydrofluorid

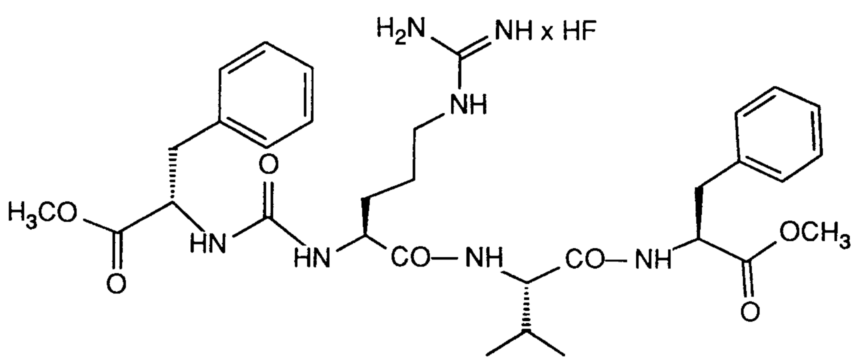

Wie für Beispiel 3 beschrieben erhält man aus 1,20 g (1,51 mmol) der Verbindung aus Beispiel 6 in Gegenwart von 1,20 g (11,10 mmol) p-Kresol und 12 ml wasserfreier Flußsäure nach Chromatographie des Rohprodukts an 20 g Kieselgel (Acetonitril : Wasser 4:1) 788 mg (79%) der Titelverbindung als farbloses Lyophilisat von pH 5,8.
$R_f$ = 0,30 (Acetonitril : Wasser 4:1)
MS (FAB) m/z = 640 $(M+H)^+$
IR (KBr) 3357, 1741, 1638, 1560, 1216, 700 $cm^{-1}$

$^1$H-NMR (250 MHz, $DMSO_{d6}$ / $D_2O$) δ = 0,82 (m, 6H, $[CH_3]_2CH$); 1,35 - 1,60 (m, 4H, $CH_2$); 1,95 (m, 1H, $[CH_3]_2CH$); 2,60 - 3,10 (m, 6H, $CH_2Ph$, $CH_2N$); 3,59 (s, 3H, $COOCH_3$); 3,62 (s, 3H, $COOCH_3$); 4,10 (m, 2H, NCHCO); 4,40 (m, 1H, NCHCO); 4,50 (m, 1H, NCHCO); 7,12 - 7,35 (m, 10H, H arom.).

Beispiel 9

(2S)-2-{$N_\alpha$-[((2S)-1-Hydroxy-3-phenyl-prop-2-yl)aminocarbonyl]-$N^G$-(4-methyl-phenylsulfonyl)-S-arginyl-S-valinyl}-amino-3-phenyl-propan-1-ol

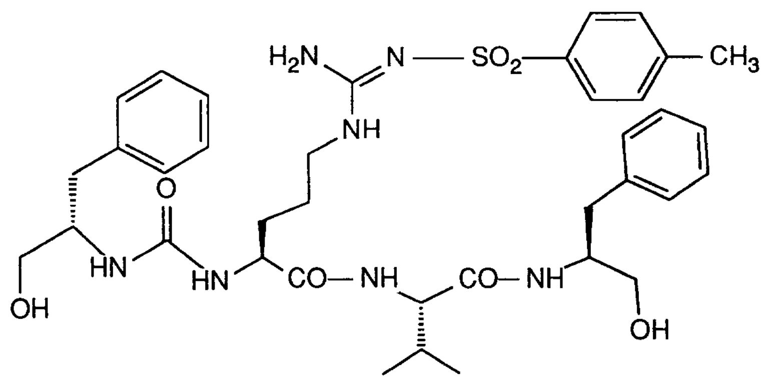

Zu einer gerührten, auf 40 °C geheizten Losung von 454 mg (12,00 mmol) Natriumborhydrid und 1,61 g (12,00 mmol) Lithiumjodid in 30 ml THF gibt man innerhalb von 10 min portionsweise 3,97 g (5,00 mmol) der Verbindung aus Beispiel 6. Zu diesem Gemisch tropft man innerhalb von 5 h bei 40 °C langsam 8 ml Methanol. Das Ende der Reaktion wird dünnschichtchromatographisch festgestellt und die Reaktionsmischung wird in 60 ml einer 10%igen Lösung von Citronensäure geschüttet. Dieses Gemisch wird mit 4 x 30 ml Ethylacetat extrahiert und die vereinigten Extrakte werden über $MgSO_4$ getrocknet. Nach Abdampfen des Lösemittels im Vakuum und Chromatographie des Rückstands an 208 g Kieselgel (Dichlormethan : Methanol 9:1) erhält man 2,11 g (57%) der Titelverbindung als farblose Kristalle.
Schmp.: 114 °C
$R_f$ = 0,31 (Dichlormethan: Methanol 85:15)
MS (FAB): m/z = 738 $(M+H)^+$
IR (KBr) 3340, 2932, 1642, 1550, 1255, 1132, 1081, 1041, 701, 676 $cm^{-1}$
$^1$H-NMR (250 MHz, $DMSO_{D6}$ / $CD_3OD$) δ = 0,78 (m, 6H, $[CH_3]_2CH$); 1,25 - 1,60 (m, 4H, $CH_2$); 1,93 (m, 1H, $[CH_3]_2CH$); 2,35 (s, 3H, $CH_3$); 2,60 - 2,90 (m, 4H, $CH_2Ph$); 3,08 (m, 2H, $CH_2N$); 3,32 (m, 4H, $CH_2O$); 3,76 (m, 1H, NCHCO); 3,97 (m, 1H, NCHCO); 4,10 (m, NCHCO unter $CD_3OD$); 7,10 - 7,35 (m, 12H, H arom.); 7,68 (d, J = 9 Hz, 2H, H arom.)

Beispiel 10

(2S)-2-{$N_\alpha$ [((2S)-1-Hydroxy-3-phenyl-prop-2-yl)aminocarbonyl]-S-arginyl-S-valinyl}amino-3-phenyl-propan-1-ol Hydrofluorid

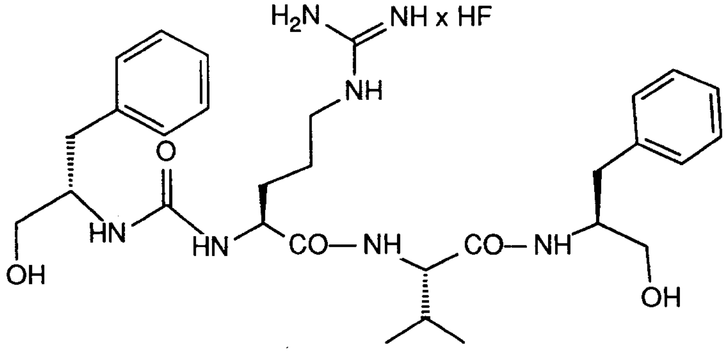

Wie für Beispiel 3 beschrieben erhält man aus 705 mg (0,96 mmol) der Verbindung aus Beispiel 9 in Gegenwart von 705 mg (6,52 mmol) p-Kresol und 10 ml wasserfreier Flußsäure nach Chromatographie des Rohprodukts an 16 g Kieselgel (Acetonitril : Wasser 7:3) 340 mg (61%) der Titelverbindung als farbloses amorphes Pulver.
$R_f$ = 0,14 (Acetonitril : Wasser 4:1)
MS (FAB): m/z = 584 $(M+H)^+$
IR (KBr) 3407, 1638, 1560, 700 $cm^{-1}$
$[\alpha]^{20}_D$ = -34,5° (c = 0,503, DMSO)
$^1$H-NMR (250 Hz, $DMSO_{d6}$ / $D_2O$): $\delta$ = 0,76 (m, 6H, $[\underline{CH_3}]_2CH$); 1,30 - 1,65 (m, 4H, $CH_2$); 1,90 (m, 1H, $[CH_3]_2\underline{CH}$); 2,62 (m, 2H, $\underline{CH_2}Ph$); 2,82 (m, 2H, $\underline{CH_2}Ph$); 3,10 (m, 2H, $CH_2N$); 3,33 (m, 4H, $CH_2O$); 3,76 (m, 1H, NCHCO); 3,92 (m, 1H unter HDO, NCHCO); 4,02 (d, J = 6,3 Hz, 1H, NCHCO-Valin); 4,12 (m, 1H, NCHCO); 7,20 (m, 10H, H arom.)

Beispiel 11

(2S)-2-{$N_\alpha$-[((1S)-1-Methoxycarbonyl-2-phenyl-ethyl)aminocarbonyl]-S-arginyl-S-valinyl}amino-3-phenyl-propan-1-ol Hydrofluorid

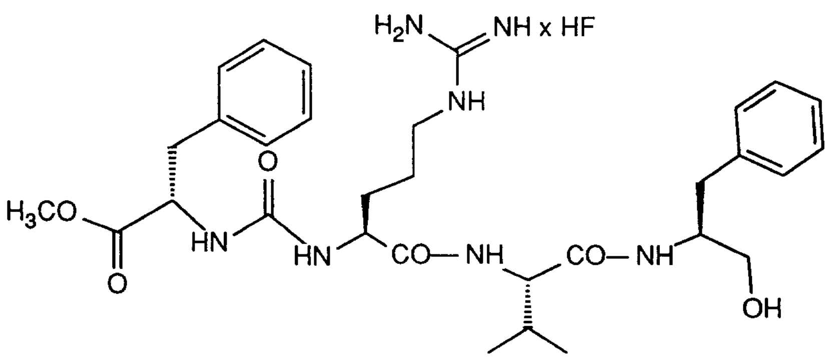

Wie für Beispiel 3 beschrieben erhalt man aus 1,30 g (1,70 mmol) der Verbindung aus Beispiel 1 in Gegenwart von 0,70 g p-Kresol und 13 ml wasserfreier Flußsäure 882 mg (82%) der Titelverbindung als farbloses Lyophilisat.
$R_f$ = 0,49 (Acetonitril : Wasser 4:1)
MS (FAB) m/z = 612 $(M+H)^+$
IR (KBr) 3422, 1736, 1654, 1560, 1216, 746 $cm^{-1}$
$[\alpha]^{20}_D$ = -55,2° (c = 0,6, DMSO)
$^1$H-NMR (300 MHz, $DMSO_{d6}/D_2O$): $\delta$ = 0,68 (m, 6H, $[\underline{CH_3}]_2CH$); 1,40 - 1,85 (m, 5H, $CH_2$, $[CH_3]_2\underline{CH}$); 2,53 (m, 1H, unter DMSO, $\underline{CH_2}Ph$); 2,80 (m, 3H, $\underline{CH_2}Ph$); 2,98 (m, 2H, $CH_2N$); 3,16 (s, 3H, $CO_2CH_3$); 3,30 (m, 2H, $CH_2O$); 3,85 (m, 2H, NCHCO); 4,41 (m, 1H, NCHCO); 7,05 - 7,30 (m, 10H, H arom.)

Beispiel 12

(2R,S)-2-{$N_\alpha$-[((1S)-1-Methoxycarbonyl-2-phenyl-ethyl)aminocarbonyl]-S-arginyl-S-valinyl}-amino-3-phenyl-propan-1-al

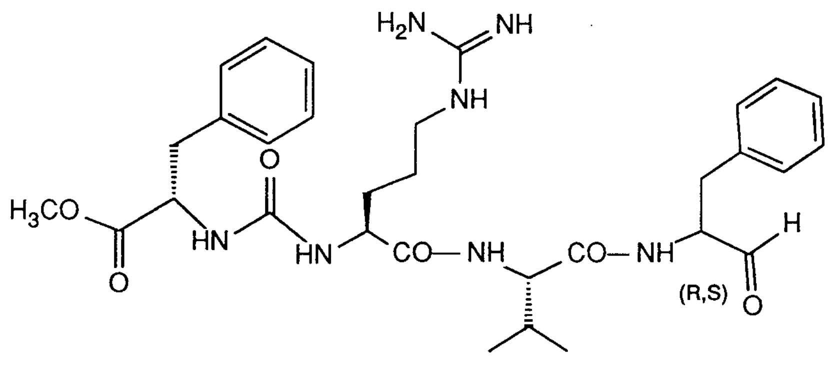

Wie für Beispiel 4 und Beispiel 5 beschrieben erhält man aus 189 mg (0,31 mmol) der Verbindung aus Beispiel 11 und 221 mg (1,39 mmol) Pyridin-Schwefeltrioxid-Komplex nach 1 h bei Raumtemperatur und Chromatographie des Rohprodukts an 45 g Kieselgel (Chloroform : Methanol : Wasser: Essigsäure 13:3:1:1)89 mg (47%) der Titelverbindung als farbloses Pulver (Gemisch der diastereomeren Aldehyde).
Schmp: ab 157 ° C (Zers.)
$R_f$ = 0,50 (Acetonitril : Wasser 4:1)
MS (FAB) m/z = 610 $(M+H)^+$
IR (KBr) 3388, 1736, 1638, 1580, 700 $cm^{-1}$
$^1$H-NMR (250 MHz, DMSO-$d_6$, $D_2O$) δ = 0,80 (m, 6H, $[CH_3]_2CH$); 1,35 - 1,60 (m, 4H, $CH_2$); 1,92 (m, 1H, $[CH_3]_2CH$]; 2,7 - 3,2 (m, 6H, $CH_2Ph$, $CH_2N$); 4,20 (m, 2H, NCHCO); 4,38 (m, 2H, NCHCO); 7,1 - 7,3 (m, 10H, H arom.); 9,45 (s, 1H, CHO).

Beispiel 13

(2R,S)-2-{$N_\alpha$-[((1S)-1-Carboxy-2-phenyl-ethyl)aminocarbonyl]-$N^G$-(4-methyl-phenylsulfonyl)-S-arginyl-S-valinyl}-amino-3-phenyl-propan-1-al

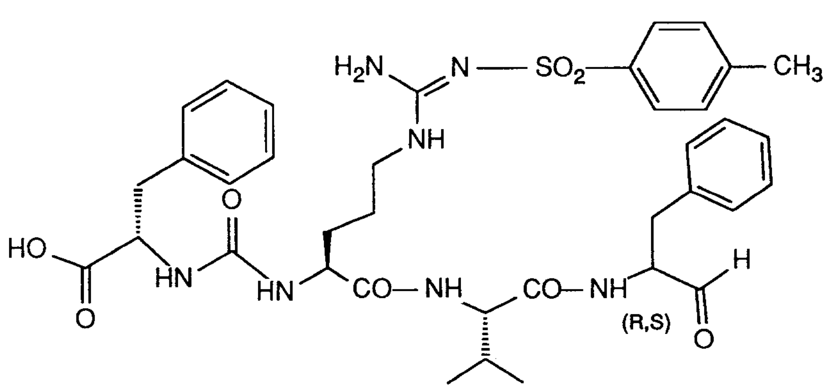

Wie für Beispiel 4 beschrieben erhält man aus 300 mg (0,40 mmol) der Verbindung aus Beispiel 2 und 286 mg (1,80 mmol) Pyridin-Schwefeltrioxid-Komplex nach 1 h bei Raumtemperatur und Chromatographie des Rohproduktes an 40 g Kieselgel (Dichlormethan : Methanol 75:25) 254 mg (85%) der Titelverbindung als farblose Kristalle (Gemisch der diastereomeren Aldehyde).
Schmp.: ab 145 ° C (Zers.)
$R_f$ = 0,33 (Dichlormethan : Methanol 7:3)
MS (FAB) m/z = 750 $(M+H)^+$

IR (KBr) 3423, 1740, 1638, 1550, 1406, 1253, 1132, 1024, 700 $cm^{-1}$
$^1$H-NMR (200 MHz, DMSO-$d_6$/$D_2O$): $\delta$ = 0,50 - 0,76 (m, 6H, $[\underline{CH_3}]_2CH$); 1,25 - 1,60 (m, 4H, $CH_2$); 1,90 (m, 1H, $[CH_3]_2\underline{CH}$); 2,35 (s, 3H, $CH_3$); 2,80 - 3,10 (m, 6H, $\underline{CH_2}Ph$, $\underline{CH_2}N$); 4,10 (m, 2H, NCHCO); 4,41 (m, 1H, NCHCO); 4,77 (m, 1H, NCHCO); 7,20 (m, 10H, H arom.); 7,30, 7,64 (AB, J = 8,5 Hz, 4H, H arom.); 9,61, 9,64 (s, 1H, CHO).

Wie in Beispiel 1 beschrieben erhält man aus der Verbindung des Beispiels V und den entsprechenden Isocyanaten ($R^1$ = NCO) die in der Tabelle 1 aufgeführten Produkte:

Tabelle 1

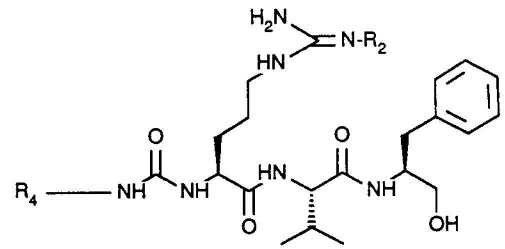

| Bsp.-Nr. | $R^2$ | $R^4$ | Ausbeute (% d.Th.) | MS (FAB) m/z $(M+H)^+$ | $R_f$ / Laufmittel-verhältnis | Schmp.: (°C) |
|---|---|---|---|---|---|---|
| 14 | $-4-SO_2-C_6H_4-CH_3$ | $-CH_2-COOC_2H_5$ | 67 | 690 | 0,25, I (9:1) | 195 |
| 15 | $-4-SO_2-C_6H_4-CH_3$ | $-CH_2CH_2COOCH(CH_3)_2$ | 95 | 718 | 0,17, I (9:1) | 229 |
| 16 | $-4-SO_2-C_6H_4-CH_3$ | $-(CH_2)_3COOCH_3$ | 73 | 704 | 0,25, I (9:1) | 159 |
| 17 | $-4-SO_2-C_6H_4-CH_3$ | $-(CH_2)_2COO-nC_4H_9$ | 76 | 732 | 0,23, I (9:1) | 120 |
| 18 | $-4-SO_2-C_6H_4-CH_3$ | $-(CH_2)_{11}CH_3$ | 72 | 772 | 0,21, I (9:1) | 151 |
| 19 | $-4-SO_2-C_6H_4-CH_3$ | $-4--C_6H_4-COOC_2H_5$ | 64 | 752 | 0,18, I (9:1) | 136 |
| 20 | $-4-SO_2-C_6H_4-CH_3$ | $-4-C_6H_4-COCH_3$ | 78 | 722 | 0,16, I (9:1) | 132 |
| 21 | $-4-SO_2-C_6H_4-CH_3$ | $-4-C_6H_4-CF_3$ | 76 | 748 | 0,20, I (9:1) | 174 |
| 22 | $-4-SO_2-C_6H_4-CH_3$ | $-2-C_6H_4-COOC_2H_5$ | 59 | 752 | 0,26, I (9:1) | 105 |

Fortsetzung Tabelle 1:

| Bsp.-Nr. | $R^2$ | $R^4$ | Ausbeute (% d.Th.) | MS (FAB) m/z $(M+H)^+$ | $R_f$ / Laufmittelverhältnis | Schmp.: (°C) |
|---|---|---|---|---|---|---|
| 23 | $-4-SO_2-C_6H_4-CH_3$ | $-4-C_6H_4-CH=CH-COOC_2H_5$ | 77 | 778 | 0,39, I (9:1) | 142 |
| 24 | $-4-SO_2-C_6H_4-CH_3$ | 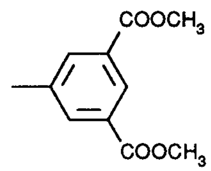 | 72 | 796 | 0,35, I (9:1) | 136 |
| 25 | $-4-SO_2-C_6H_4-CH_3$ | $3-C_6H_4-COOCH_2CH(CH_3)_2$ | 83 | 780 | 0,29, I (9:1) | 115 |
| 26 | $-4-SO_2-C_6H_4-CH_3$ | $3-C_6H_4-COO(CH_2)_3CH_3$ | 89 | 780 | 0,37, I (9:1) | 114 |
| 27 | $-4-SO_2-C_6H_4-CH_3$ | $4-C_6H_4-COO(CH_2)_3CH_3$ | 87 | 780 | 0,36, I (9:1) | 128 |
| 28 | $-4-SO_2-C_6H_4-CH_3$ | $3-C_6H_4-COOC_2H_5$ | 62 | 752 | 0,23, I (9:1) | 112 |
| 29 | $-4-SO_2-C_6H_4-CH_3$ | $3-C_6H_4-COOCH(CH_3)_2$ | 56 | 766 | 0,44, I (9:1) | 145 |
| 30 | $-4-SO_2-C_6H_4-CH_3$ | $-C(CH_3)_3$ | 59 | 660 | 0,43, I (9:1) | 130 |
| 31 | 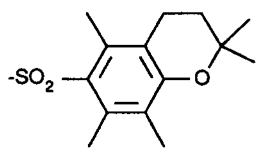 | $-(CH_2)_2COOCH(CH_3)_2$ | 56 | 830 | 0,51, I (9:1) | 198 |
| 32 | 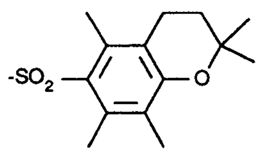 | $-4-C_6H_4-COOC_2H_5$ | 52 | 864 | 0,61, I (87:13) | 214 |

Fortsetzung Tabelle 1:

| Bsp.-Nr. | $R^2$ | $R^4$ | Ausbeute (% d.Th.) | MS (FAB) m/z $(M+H)^+$ | $R_f$ / Laufmittel-verhältnis | Schmp.: (°C) |
|---|---|---|---|---|---|---|
| 33 | 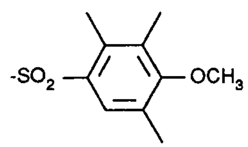 | $-(CH_2)_2COOCH(CH_3)_2$ | 55 | 776 | 0,37, I (9:1) | 114 |
| 34 | 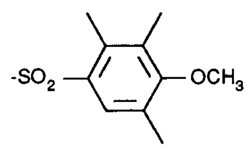 | $-4-C_6H_4-COOC_2H_5$ | 59 | 810 | 0,40, I (87:13) | 218 |
| 34a | $-4-SO_2-C_6H_4-CH_3$ | $-CH_2C(CH_3)_2OCH_3$ | 67 | 690 | 0,14, I (9:1) | 162 |

Wie für Beispiel 2 beschrieben erhält man durch Verseifung der Verbindungen (Startmaterial) aus den angegebenenen Beispielen die in der Tabelle 2 aufgeführten Produkte:

Tabelle 2

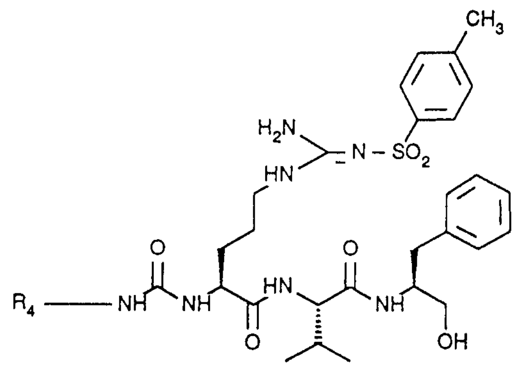

| Bsp.-Nr. | $R^4$ | Ausbeute (% d.Th.) | MS (FAB) m/z $(M+H)^+$ | $R_f$ / Laufmittelverhältnis | Schmp.: (°C) | IR $\gamma$ ($cm^{-1}$) | Startmaterial aus Beispiel-Nr. |
|---|---|---|---|---|---|---|---|
| 35 | $-CH_2-COOH$ | 83 | 662 | 0,16, III (9:1) | 158 | 1727, 1641, 1551, 1248 | 14 |
| 36 | $-CH_2CH_2COOH$ | 82 | 676 | 0,22, III (9:1) | 157 | 1728, 1621, 1552, 1274 | 15 |

Wie für Beispiel 3 beschrieben erhält man durch Umsetzung der entsprechenden Startmaterialien in wasserfreier Flußsäure die in Tabelle 3 aufgeführten Produkte:

Tabelle 3:

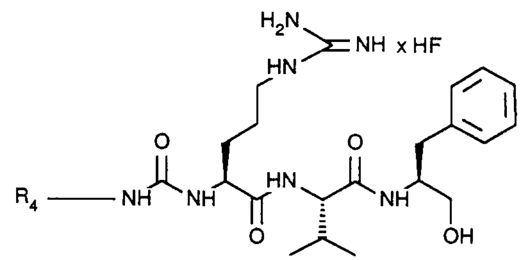

| Bsp.-Nr. | $R^4$ | Ausbeute (% d.Th.) | MS (FAB) m/z $(M+H)^+$ | $R_f$ / Laufmittel-verhältnis | $[\alpha]^{20}_D$ (c, DMSO) | IR $\gamma$ ($cm^{-1}$) |
|---|---|---|---|---|---|---|
| 37 | $-CH_2COOH$ | 93 | 508 | 0,33, III (7:3) | -22,5 (0,96) | 1638, 1560, 1204, 1137 |
| 38 | $-CH_2COOC_2H_5$ | 97 | 536 | 0,35, III (85:15) | -20.8 (0,596) | 1654, 1560, 1205 |
| 39 | $-CH_2CH_2COOH$ | 98 | 522 | 0,24, III (7:3) | -18,5 (0,486) | 1654, 1560, 1203 |
| 40 | $-CH_2CH_2COOCH(CH_3)_2$ | 62 | 564 | 0,53, III (7:3) | -16,2 (0,362) | 1655, 1560 1203 |

Wie für Beispiel 4 und 5 beschrieben erhalt man durch Oxidation der entsprechenden Startmaterialien mit Pyridin-$SO_3$ in DMSO und gegebenenfalls Trennung der Diastereomere durch Chromatographie die in der Tabelle 4 aufgeführten Verbindungen:

Tabelle 4:

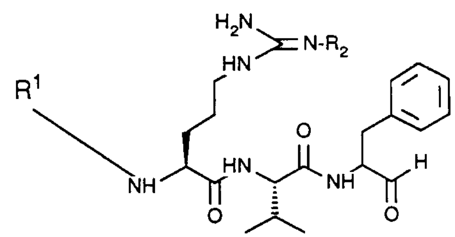

| Bsp.-Nr. | $R^1$ | $R^2$ | Ausbeute (% d.Th.) | MS (FAB) m/z $(M+H)^+$ | $R_f$ / Laufmittel-verhältnis | Diastereomer | Smp. (°C) |
|---|---|---|---|---|---|---|---|
| 41 | $-CO-NH-CH_2COOH$ | H | 27 | 506 | 0,32, III (7:3) | unpolar, polar | 203 |
| 42 | $-CO-NH-CH_2\,C(CH_3)_2OCH_3$ | $-4-SO_2C_6H_4-CH_3$ | 69 | 688 | 0,30; 0,33, I (9:1) | unpolar, polar | 159 |
| 43 | $-CO-NH-(CH_2)_2COOCH(CH_3)_2$ | $-4-SO_2-C_6H_4-CH_3$ | 27 | 716 | 0,29, I (9:1) | polar | 143 a) |
| 44 | $-CO-NH-(CH_2)_2COOCH(CH_3)_2$ | $-4-SO_2-C_6H_4-CH_3$ | 37 | 716 | 0,26, I (9:1) | unpolar | 135 b) |
| 45 | $-CO-NH-CH_2CH_2COOH$ | $-4-SO_2-C_6H_4-CH_3$ | 44 | 674 | 0,30, III (9:1) | unpolar, polar | – |
| 46 | $-CO-NH-CH_2CH_2COOH$ | $-4-SO_2-C_6H_4-CH_3$ | 53 | 520 | 0,26, III (7:3) | unpolar, polar | - |
| 47 | $-CO-NH-CH_2-COOC_2H_5$ | $-4-SO_2-C_6H_4-CH_3$ | 30 | 688 | 0,26, I (9:1) | unpolar | 109 |
| 48 | $-CO-NH-CH_2-COOC_2H_5$ | $-4-SO_2-C_6H_4-CH_3$ | 23 | 688 | 0,22, I (9:1) | polar | 124 |

a) unpolares Isomer: $[\alpha]^{20}_D$ = 19,5° (c=0,85, DMSO)
b) polares Isomer: $[\alpha]^{20}_D$ = -16,4° (c=0,85, DMSO)

Fortsetzung Tabelle 4:

| Bsp.-Nr. | $R^1$ | $R^2$ | Ausbeute (% d.Th.) | MS (FAB) m/z $(M+H)^+$ | $R_f$ / Laufmittel-verhältnis | Diastereomer | Smp. (°C) |
|---|---|---|---|---|---|---|---|
| 49 | $-CO-NH-(CH_2)_3COOCH_3$ | $-4-SO_2-C_6H_4-CH_3$ | 24 | 702 | 0,29, I (9:1) | unpolar | 134 |
| 50 | $-CO-NH-(CH_2)_3COOCH_3$ | $-4-SO_2-C_6H_4-CH_3$ | 21 | 702 | 0,27, I (9:1) | polar | 156 |
| 51 | $-CO-NH-(CH_2)_2-COO-n-C_4H_9$ | $-4-SO_2-C_6H_4-CH_3$ | 18 | 730 | 0,20, I (9:1) | unpolar | 122 |
| 52 | $-CO-NH-(CH_2)_2-COO-n-C_4H_9$ | $-4-SO_2-C_6H_4-CH_3$ | 23 | 730 | 0,16, I (9:1) | polar | 134 |
| 53 | $-CO-NH-(CH_2)_{11}-CH_3$ | $-4-SO_2-C_6H_4-CH_3$ | 20 | 770 | 0,14, I (9:1) | unpolar | 140 |
| 54 | $-CO-NH-(CH_2)_{11}-CH_3$ | $-4-SO_2-C_6H_4-CH_3$ | 23 | 770 | 0,11, I (9:1) | polar | 122 |
| 55 | $-CO-NH-4-C_6H_4-COOC_2H_5$ | $-4-SO_2-C_6H_4-CH_3$ | 30 | 750 | 0,26, I (9:1) | unpolar | 182 |
| 56 | $-CO-NH-4-C_6H_4-COOC_2H_5$ | $-4-SO_2-C_6H_4-CH_3$ | 39 | 750 | 0,21, I (9:1) | polar | 164 |
| 57 | $-CO-NH-4-C_6H_4-COCH_3$ | $-4-SO_2-C_6H_4-CH_3$ | 32 | 720 | 0,16, I (9:1) | unpolar | 122 |
| 58 | $-CO-NH-4-C_6H_4-COCH_3$ | $-4-SO_2-C_6H_4-CH_3$ | 31 | 720 | 0,14, I (9:1) | polar | 102 |

Fortsetzung Tabelle 4:

| Bsp.-Nr. | $R^1$ | $R^2$ | Ausbeute (% d.Th.) | MS (FAB) m/z $(M+H)^+$ | $R_f$ / Laufmittel-verhältnis | Diastereomer | Smp. (°C) |
|---|---|---|---|---|---|---|---|
| 59 | $-CO-NH-C(CH_3)_3$ | $-4-SO_2-C_6H_4-CH_3$ | 58 | 658 | 0,21; 0,24, I (9:1) | unpolar, polar | 110 |
| 60 | $-CO-NH-3,5-C_6H_3-CO_2CH_3$ | $-4-SO_2-C_6H_4-CH_3$ | 67 | 794 | 0,21; 0,24, I (9:1) | unpolar, polar | 122 |
| 61 | $-CO-NH-4-C_6H_4-CF_3$ | $-4-SO_2-C_6H_4-CH_3$ | 63 | 746 | 0,14; 0,17, I (9:1) | unpolar, polar | 165 |
| 62 | $-CO-NH-4-C_6H_4-CH=CH-COOC_2H_5$ | $-4-SO_2-C_6H_4-CH_3$ | 83 | 776 | 0,27; 0,29, I (9:1) | unpolar, polar | 128 |
| 63 | $-CO-NH-2-C_6H_4-COOC_2H_5$ | $-4-SO_2-C_6H_4-CH_3$ | 76 | 750 | 0,14; 0,18, I (9:1) | unpolar, polar | 115 |
| 64 | $-CO-NH-3-C_6H_4-COOC_2H_5$ | $-4-SO_2-C_6H_4-CH_3$ | 49 | 750 | 0,18; 0,22, I (9:1) | unpolar, polar | 136 |
| 65 | $-CO-NH-3-C_6H_4.COOCH(CH_3)_2$ | $-4-SO_2-C_6H_4-CH_3$ | 52 | 764 | 0,52; 0,57, I (9:1) | unpolar, polar | 124 |
| 66 | $-CO-NH-3-C_6H_4-COOCH_2CH(CH_3)_2$ | $-4-SO_2-C_6H_4-CH_3$ | 85 | 778 | 0,38; 0,44, I (9:1) | unpolar, polar | 108 |
| 67 | $-CO-NH-3-C_6H_4-COO(CH_2)_3CH_3$ | $-4-SO_2-C_6H_4-CH_3$ | 84 | 778 | 0,24; 0,28, I (9:1) | unpolar, polar | 106 |
| 68 | $-CO-NH-4-C_6H_4-COO(CH_2)_3CH_3$ | $-4-SO_2-C_6H_4-CH_3$ | 85 | 778 | 0,23; 0,32, I (9:1) | unpolar, polar | - |

Fortsetzung Tabelle 4:

| Bsp.-Nr. | $R^1$ | $R^2$ | Ausbeute (% d.Th.) | MS (FAB) m/z $(M+H)^+$ | $R_f$ / Laufmittel-verhältnis | Diastereomer | Smp. (°C) |
|---|---|---|---|---|---|---|---|
| 69 | Boc | $-4-SO_2-C_6H_4-CH_3$ | 31 | 659 | 0,26, I (9:1) | unpolar | 138 |
| 70 | Boc | $-4-SO_2-C_6H_4-CH_3$ | 53 | 659 | 0,24, I (9:1) | polar | Schaum |
| 71 | Fmoc- | 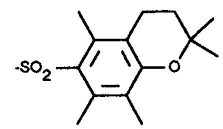 | 10 | 893 | 0,30, I (95:5) | unpolar | 131 |
| 72 | Fmoc- | 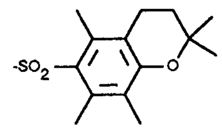 | 5 | 893 | 0,22, I (95:5) | polar | 132 |
| 73 | Fmoc- | 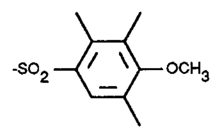 | 11 | 839 | 0,39, I (85:15) | unpolar | 153 |
| 74 | Fmoc- | 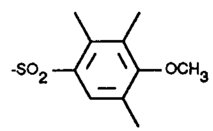 | 10 | 839 | 0,33, I (85:15) | polar | 92 |
| 75 | $PhCH_2OCO-$ | $-4-SO_2-C_6H_4-CH_3$ | 65 | 693 | 0,44; 0,52, I (9:1) | unpolar, polar | Öl |

Fortsetzung Tabelle 4:

| Bsp.-Nr. | $R^1$ | $R^2$ | Ausbeute (% d.Th.) | MS (FAB) m/z $(M+H)^+$ | $R_f$ / Laufmittel-verhältnis | Diastereomer | Smp. (°C) |
|---|---|---|---|---|---|---|---|
| 76 | $-CO-NH-(CH_2)_2COOCH(CH_3)_2$ | 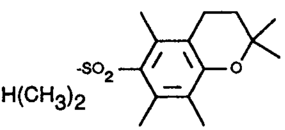 | 70 | 828 | 0,17; 0,24, I (87:13) | unpolar, polar | 142 |
| 77 | $-CO-NH-4-C_6H_4-COOC_2H_5$ | 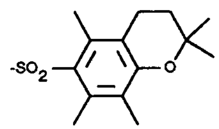 | 79 | 862 | 0,22; 0,26, I (9:1) | unpolar, polar | 114 |
| 78 | $-CO-NH-CH_2)_2COOCH(CH_3)_2$ | 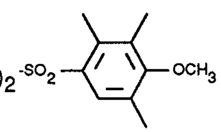 | 64 | 775 | 0,24; 0,26, I (87:13) | unpolar, polar | 153 |
| 79 | $-CO-NH-4-C_6H_4-COOC_2H_5$ | 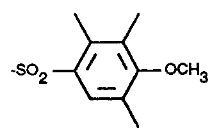 | 79 | 808 | 0,18; 0,22, I (9:1) | unpolar, polar | 138 |
| 80 | $CH_3CO-$ | 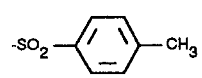 | 59 | 601 | 0,26; 0,30, I (9:1) | unpolar, polar | 104 |
| 81 | $-CO-NH-CH_2-C(CH_3)_2OCH_3$ | 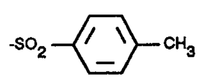 | 69 | 688 | 0,30; 0,33, I (9:1) | unpolar, polar | 159 |

Wie für Beispiel IV beschrieben erhält man durch Kondensation der Verbindung aus Beispiel III mit dem entsprechenden S-Argininderivat die in der Tabelle 5 aufgeführten Verbindungen:

Tabelle 5:

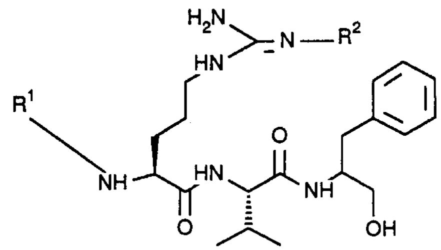

| Bsp.-Nr. | $R^2$ | $R^1$ | Ausbeute (% d.Th.) | MS (FAB) m/z $(M+H)^+$ | $R_f$ / Laufmittel-verhältnis | Schmp.: (°C) |
|---|---|---|---|---|---|---|
| 82 | $-4-SO_2-C_6H_4-CH_3$ | $Ph-CH_2-O-CO$ | 50 | 695 | 0,34.,I (9:1) | 110 |
| 83 | 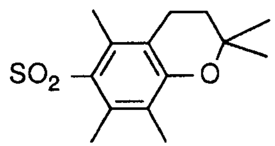 | Fmoc | 57 | 895 | 0,35, II (0:10) | 138 |
| 84 | 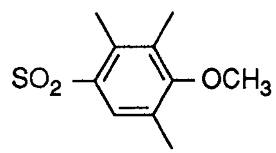 | Fmoc | 55 | 841 | 0,58, I (85:15) | 179 |

Beispiel 85

(2S)-2-[$N_\alpha$-Acetyl-$N^G$-(4-methyl-phenylsulfonyl)-S-arginyl-S-valinyl-amino-3-phenyl-propan-1-ol

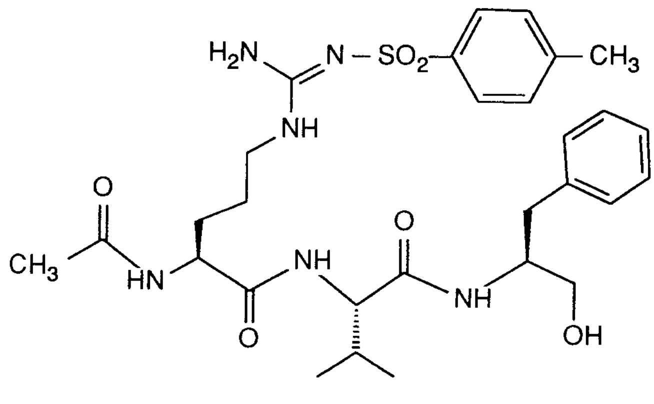

Zu einer auf 0°C gekühlten Lösung von 951 mg (150 mmol) der Verbindung aus Beispiel V in 6 ml wasserfreiem Dimethylformamid und 660 µl (6,00 mmol) N-Methylmorpholin werden 180 µl (1,86 mmol) Essigsäureanhydrid getropft. Nach 15 min bei 0°C wird in ein Gemisch aus 120 ml eiskalter Natriumbicarbonat-Lösung und 60 ml Ethylacetat gegossen und gut durchgerührt. Die organische Phase wird abgetrennt, die Wasserphase mit 10 ml Ethylacetat extrahiert und die vereinigten organischen Extrakte über Magnesiumsulfat getrocknet. Nach Abdampfen des Lösemittels wird der Rückstand durch verreiben in 10 ml Dichlormethan und 50 ml Ether kristallisiert. Man erhält 532 mg (59 %) der Titelverbindung als farblose Kristalle.
Schmp.: 160-162°C
$R_f$ = 0,10 Dichlormethan:Methanol 9:1
MS (FAB) m/z = 603 $(M+H)^+$

**Patentansprüche**

1. Pseudopeptide der allgemeinen Formel (I)

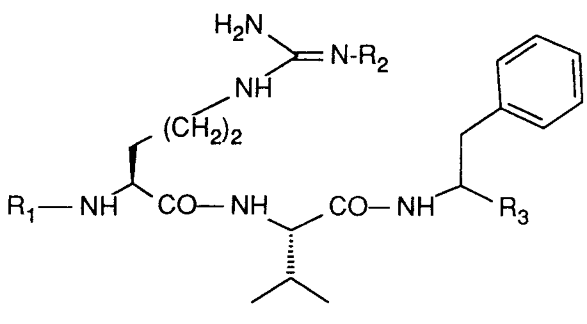

(I)

in welcher

| | |
|---|---|
| $R^1$ | für eine Aminoschutzgruppe steht, oder für einen Rest der Formel $R^4$-NH-CO- steht, worin |
| $R^4$ | Cycloalkyl mit 3 bis 6 Kohlenstoffatomen bedeutet oder geradkettiges oder verzweigtes Alkyl mit bis zu 18 Kohlenstoffatomen bedeutet, das gegebenenfalls durch Hydroxy, geradkettiges oder verzweigtes Alkoxy mit bis zu 4 Kohlenstoffatomen, Halogen, Trifluormethyl, Trifluormethoxy, Cycloalkyl mit 3 bis 6 Kohlenstoffatomen oder durch Aryl mit 6 bis 10 Kohlenstoffatomen substituiert ist, das seinerseits bis zu 2-fach gleich oder verschieden durch Carboxy, Cyano, Hydroxy, Halogen, Perhalogenalkyl mit bis zu 5 Kohlenstoffatomen oder durch geradkettiges oder verzweigtes Acyl, Alkoxy oder Alkoxycarbonyl mit jeweils bis zu 6 Kohlenstoffatomen substituiert sein kann, oder |

Alkyl gegebenenfalls durch eine Gruppe der Formel $-CO_2R^5$ substituiert ist,
worin

$R^5$ Wasserstoff oder geradkettiges oder verzweigtes Alkyl oder Alkenyl mit jeweils bis zu 8 Kohlenstoffatomen bedeutet, die gegebenenfalls durch Phenyl substituiert sind,
oder

$R^4$ Aryl mit 6 bis 10 Kohlenstoffatomen bedeutet, das gegebenenfalls bis zu 3-fach gleich oder verschieden durch Carboxy, Halogen, Hydroxy, Cyano, Perhalogenalkyl mit bis zu 5 Kohlenstoffatomen oder durch geradkettiges oder verzweigtes Acyl, Alkoxy, Alkoxycarbonyl oder Vinylalkoxycarbonyl mit jeweils bis zu 6 Kohlenstoffatomen substituiert ist, oder
einen Aminosäurerest der Formel

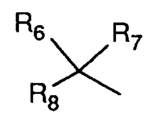

bedeutet,
worin

$R^6$ und $R^7$ gleich oder verschieden sind und Wasserstoff oder Methyl bedeuten, oder

$R^6$ und $R^7$ gemeinsam einen 5- oder 6-gliedrigen gesättigten carbocyclischen Ring bilden,
oder

$R^6$ Wasserstoff oder Methyl bedeutet
und

$R^7$ Cycloalkyl mit 3 bis 8 Kohlenstoffatomen oder Aryl mit 6 bis 10 Kohlenstoffatomen oder Wasserstoff bedeutet, oder
geradkettiges oder verzweigtes Alkyl mit bis zu 8 Kohlenstoffatomen bedeutet,
wobei das Alkyl gegebenenfalls durch Methylthio, Hydroxy, Mercapto,
Guanidyl oder durch eine Gruppe der Formel $-NR^9R^{10}$ oder $R^{11}$-OC- substituiert ist,
worin

$R^9$ und $R^{10}$ unabhängig voneinander für Wasserstoff, geradkettiges oder verzweigtes Alkyl mit bis zu 8 Kohlenstoffatomen oder Phenyl stehen,
und

$R^{11}$ Hydroxy, Benzyloxy, Alkoxy mit bis zu 6 Kohlenstoffatomen oder die oben aufgeführte Gruppe $-NR^9R^{10}$ bedeutet,

oder das Alkyl gegebenenfalls durch Cycloalkyl mit 3 bis 8 Kohlenstoffatomen oder durch Aryl mit 6 bis 10 Kohlenstoffatomen substituiert ist, das seinerseits durch Hydroxy, Halogen, Nitro, Alkoxy mit bis zu 8 Kohlenstoffatomen oder durch die Gruppe $-NR^9R^{10}$ substituiert ist,
worin

$R^9$ und $R^{10}$ die oben angegebene Bedeutung haben,

oder das Alkyl gegebenenfalls durch einen 5- bis 6-gliedrigen stickstoffhaltigen Heterocyclus oder Indolyl substituiert ist, worin die entsprechenden -NH-Funktionen gegebenenfalls durch Alkyl mit bis zu 6 Kohlenstoffatomen oder durch eine Aminoschutzgruppe geschützt sind,

$R^8$ geradkettiges oder verzweigtes Alkyl mit bis zu 8 Kohlenstoffatomen bedeutet, das gegebenenfalls durch Hydroxy oder geradkettiges oder verzweigtes Alkoxy mit bis zu 6 Kohlenstoffatomen substituiert ist, oder
Carboxy, Allyloxycarbonyl, geradkettiges oder verzweigtes Alkoxycarbonyl mit bis zu 8 Kohlenstoffatomen oder Benzyloxycarbonyl bedeutet,

$R^2$ für Wasserstoff, für eine Aminoschutzgruppe oder für einen Rest der Formel $-SO_2-R^{12}$ steht,
worin

$R^{12}$ Methyl oder Phenyl bedeutet, das gegebenenfalls bis zu 4-fach gleich oder verschieden durch Methyl oder Methoxy substitiert ist, oder
einen Rest der Formel

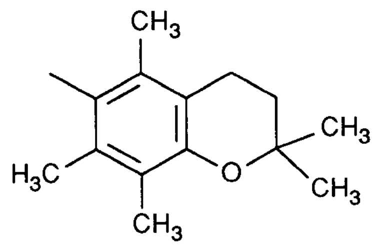

bedeuten,

R³ — für Formyl oder Carboxy steht oder für geradkettiges oder verzweigtes Alkoxycarbonyl mit bis zu 8 Kohlenstoffatomen steht oder für einen Rest der Formel $-CH_2-OR^{13}$ oder $-CH(OR^{14})_2$ steht, worin

$R^{13}$ und $R^{14}$ — gleich oder verschieden sind und Wasserstoff, geradkettiges oder verzweigtes Alkyl mit bis zu 6 Kohlenstoffatomen oder eine Hydroxyschutzgruppe bedeuten, gegebenenfalls in einer isomeren Form, und deren Salze, mit der Maßgabe, daß

$R^4$ — nicht den Rest der Formel

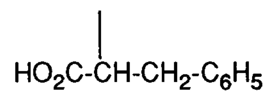

bedeuten darf, wenn $R^2$ für Wasserstoff und $R^3$ entweder für Formyl oder für Carboxy steht, und

$R^4$ — nicht den Rest der Formel

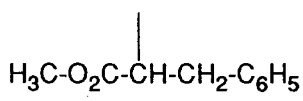

bedeuten darf, wenn $R^2$ für Wasserstoff und $R^3$ für die $-CH(OCH_3)_2$-Gruppe steht.

**2.** Verbindungen der allgemeinen Formel (I), gemäß Anspruch 1 in welcher

$R^1$ — für Acetyl, tert.Butoxycarbonyl (Boc), Benzyloxycarbonyl (Z) oder 9-Fluorenylmethoxycarbonyl (Fmoc) steht, oder für einen Rest der Formel $R^4$-NH-CO- steht, worin

$R^4$ — Cyclopentyl oder Cyclohexyl bedeutet oder geradkettiges oder verzweigtes Alkyl mit bis zu 16 Kohlenstoffatomen bedeutet, das gegebenenfalls durch Hydroxy, Methoxy, Fluor, Chlor, Brom, Trifluormethyl, Trifluormethoxy, Cyclopentyl, Cyclohexyl oder Phenyl substituiert ist, das seinerseits bis zu 2-fach gleich oder verschieden durch Carboxy, Cyano, Hydroxy, Fluor, Chlor, Brom, Perhalogenalkyl mit bis zu 4 Kohlenstoffatomen oder durch geradkettiges oder verzweigtes Acyl, Alkoxy oder Alkoxycarbonyl mit jeweils bis zu 4 Konlenstoffatomen substituiert sein kann, oder Alkyl gegebenenfalls durch eine Gruppe der Formel $-CO_2R^5$ substituiert ist, worin

$R^5$ — Wasserstoff oder geradkettiges oder verzweigtes Alkyl oder Alkenyl mit jeweils bis zu 6 Kohlenstoffatomen bedeutet, die gegebenenfalls durch Phenyl substituiert sind, oder

$R^4$ — Phenyl oder Naphthyl bedeutet, das gegebenenfalls bis zu 3-fach gleich oder verschieden durch Carboxy, Fluor, Chlor, Brom, Hydroxy, Cyano, Perhalogenalkyl

mit bis zu 4 Kohlenstoffatomen oder durch geradkettiges oder verzweigtes Acyl, Alkoxy, Alkoxycarbonyl oder Vinylalkoxycarbonyl mit jeweils bis zu 5 Kohlenstoffatomen substituiert ist, oder
einen Aminosäurerest der Formel

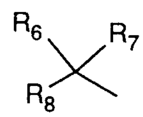

bedeutet,
worin

$R^6$ und $R^7$ gleich oder verschieden sind und Wasserstoff oder Methyl bedeuten, oder

$R^6$ und $R^7$ gemeinsam einen Cyclopentyl- oder Cyclohexylring bilden,
oder

$R^6$ Wasserstoff oder Methyl bedeutet
und

$R^7$ Cyclopentyl, Cyclohexyl, Phenyl oder Wasserstoff bedeutet, oder
geradkettiges oder verzweigtes Alkyl mit bis zu 6 Kohlenstoffatomen bedeutet, wobei das Alkyl gegebenenfalls durch Methylthio, Hydroxy, Mercapto, Guanidyl, Amino, Carboxy oder $H_2N$-CO- substituiert sein kann,
oder das Alkyl durch Cyclohexyl, Naphtyl oder Phenyl substituiert ist, das seinerseits durch Fluor, Hydroxy, Nitro, Alkoxy mit bis zu 4 Kohlenstoffatomen substituiert sein kann,
oder das Alkyl gegebenenfalls durch Indolyl, Imidazolyl, Pyridyl, Triazolyl oder Pyrazolyl substituiert ist, wobei die entsprechenden -NH-Funktionen gegebenenfalls durch Alkyl mit bis zu 4 Kohlenstoffatomen oder durch eine Aminoschutzgruppe geschützt sind,

$R^8$ geradkettiges oder verzweigtes Alkyl mit bis zu 6 Kohlenstoffatomen bedeutet, das gegebenenfalls durch Hydroxy oder geradkettiges oder verzweigtes Alkoxy mit bis zu 4 Kohlenstoffatomen substituiert ist, oder Carboxy, Allyloxycarbonyl, geradkettiges oder verzweigtes Alkoxycarbonyl mit bis zu 6 Kohlenstoffatomen oder Benzyloxycarbonyl bedeutet,

$R^2$ für Wasserstoff, Benzyloxycarbonyl, tert.Butoxycarbonyl oder für einen Rest der Formel $-SO_2-R^{12}$ steht,
worin

$R^{12}$ Methyl oder Phenyl bedeutet, das gegebenenfalls bis zu 4-fach gleich oder verschieden durch Methyl oder Methoxy substituiert ist, oder
einen Rest der Formel

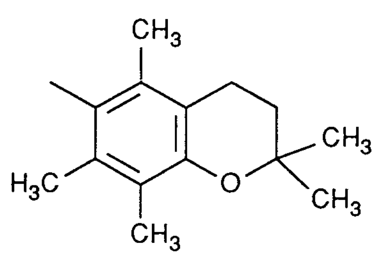

bedeutet,

$R^3$ für Formyl oder Carboxy steht oder
für geradkettiges oder verzweigtes Alkoxycarbonyl mit bis zu 6 Kohlenstoffatomen steht, oder
für einen Rest der Formel $-CH_2-OR^{13}$ oder $-CH(OR^{14})_2$ steht,
worin

$R^{13}$ und $R^{14}$ gleich oder verschieden sind und Wasserstoff, geradkettiges oder verzweigtes Alkyl

mit bis zu 4 Kohlenstoffatomen, Acetyl oder Benzyl bedeuten, gegebenenfalls in einer isomeren Form, und deren Salze, mit der Maßgabe, daß

$R^4$ nicht den Rest der Formel

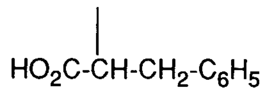

bedeuten darf, wenn $R^2$ für Wasserstoff und $R^3$ entweder für Formyl oder für Carboxy steht, und

$R^4$ nicht den Rest der Formel

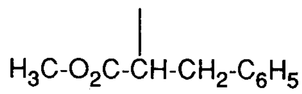

bedeuten darf, wenn $R^2$ für Wasserstoff und $R^3$ für die $-CH(OCH_3)_2$-Gruppe steht.

**3.** Verbindungen der allgemeinen Formel (I), gemäß Anspruch 1 in welcher

$R^1$ für Acetyl, tert.Butoxycarbonyl (Boc), Benzyloxycarbonyl (Z) oder 9-Fluorenylmethoxycarbonyl (Fmoc) steht, oder für einen Rest der Formel $R^4$-NH-CO- steht, worin

$R^4$ Cyclopentyl oder Cyclohexyl bedeutet oder geradkettiges oder verzweigtes Alkyl mit bis zu 14 Kohlenstoffatomen bedeutet, das gegebenenfalls durch Hydroxy, Methoxy, Fluor, Trifluormethyl, Trifluormethoxy, Cyclohexyl oder Phenyl substituiert ist, oder das gegebenenfalls durch eine Gruppe der Formel $-CO_2R^5$ substituiert ist, worin

$R^5$ Wasserstoff oder geradkettiges oder verzweigtes Alkyl oder Alkenyl mit jeweils bis zu 4 Kohlenstoffatomen oder Benzyl bedeutet, oder

$R^4$ Phenyl bedeutet, das gegebenenfalls bis zu 2-fach gleich oder verschieden durch Carboxy, Fluor, Hydroxy, Cyano, Trifluormethyl oder durch geradkettiges oder verzweigtes Acyl, Alkoxy, Alkoxycarbonyl oder Vinylalkoxycarbonyl mit jeweils bis zu 4 Kohlenstoffatomen substituiert ist, oder einen Aminosäurerest der Formel

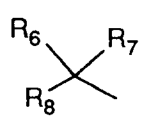

bedeutet, worin

$R^6$ und $R^7$ gleich oder verschieden sind und Wasserstoff oder Methyl bedeuten, oder

$R^6$ und $R^7$ gemeinsam einen Cyclohexylring bilden, oder

$R^6$ Wasserstoff oder Methyl bedeutet und

$R^7$ Cyclopentyl, Cyclohexyl oder Wasserstoff bedeutet, oder geradkettiges oder verzweigtes Alkyl mit bis zu 4 Kohlenstoffatomen bedeutet, wobei das Alkyl gegebenenfalls durch Methylthio, Hydroxy, Mercapto, Guanidyl, Amino, Carboxy oder $H_2N$-CO- substituiert sein kann,

oder das Alkyl durch Cyclohexyl, Naphthyl oder Phenyl substituiert ist, das

seinerseits durch Fluor, Chlor oder Alkoxy mit bis zu 4 Kohlenstoffatomen substituiert sein kann,

oder das Alkyl gegebenenfalls durch Imidazolyl, Pyridyl oder Pyrazolyl substituiert ist, wobei die entsprechenden -NH-Funktionen gegebenenfalls durch Methyl, Benzyloxymethylen oder tert.Butyloxycarbonyl (Boc) geschützt sind,

$R^8$ geradkettiges oder verzweigtes Alkyl mit bis zu 4 Kohlenstoffatomen bedeutet, das gegebenenfalls durch Hydroxy oder geradkettiges oder verzweigtes Alkoxy mit bis zu 3 Kohlenstoffatomen substituiert ist, oder

Carboxy, Allyloxycarbonyl, geradkettiges oder verzweigtes Alkoxycarbonyl mit bis zu 4 Kohlenstoffatomen oder Benzyloxycarbonyl bedeutet,

$R^2$ für Wasserstoff, oder

für einen Rest der Formel $-SO_2-R^{12}$ steht,

worin

$R^{12}$ Methyl oder Phenyl bedeutet, das gegebenenfalls bis zu 4-fach gleich oder verschieden durch Methyl oder Methoxy substitiert ist, oder

einen Rest der Formel

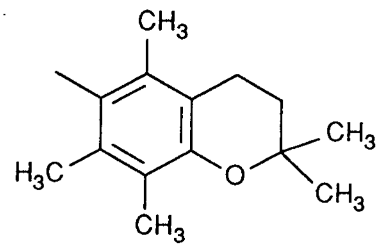

bedeutet,

$R^3$ für Formyl oder Carboxy steht oder

für geradkettiges oder verzweigtes Alkoxycarbonyl mit bis zu 4 Kohlenstoffatomen steht, oder

für einen Rest der Formel $-CH_2-OR^{13}$ oder $-CH(OR^{14})_2$ steht,

worin

$R^{13}$ und $R^{14}$ gleich oder verschieden sind und Wasserstoff, geradkettiges oder verzweigtes Alkyl mit bis zu 3 Kohlenstoffatomen oder Benzyl bedeuten,

gegebenenfalls in einer isomeren Form, und deren Salze,

mit der Maßgabe, daß

$R^4$ nicht den Rest der Formel

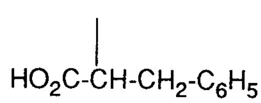

bedeuten darf, wenn $R^2$ für Wasserstoff und $R^3$ entweder für Formyl oder für Carboxy steht,

und

$R^4$ nicht den Rest der Formel

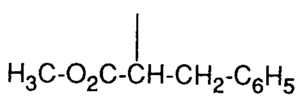

bedeuten darf, wenn $R^2$ für Wasserstoff und $R^3$ für die $-CH(OCH_3)_2$-Gruppe steht.

**4.** Verfahren zur Herstellung der Verbindungen der allgemeinen Formel (I), gemäß Anspruch 1, dadurch gekennzeichnet, daß man

Verbindungen der allgemeinen Formel (III)

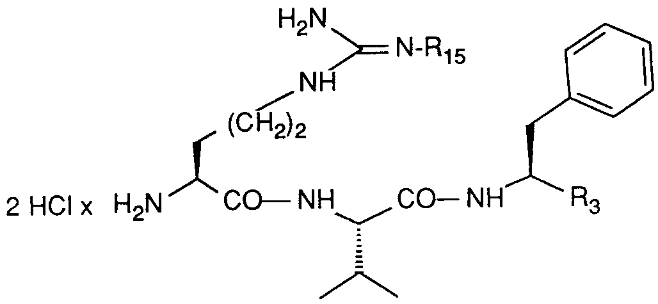

(III)

in welcher

$R^3$ die oben angegebene Bedeutung hat

und

$R^{15}$ die oben angegebenen Bedeutung von $R^2$ hat, aber nicht für Wasserstoff steht

zunächst durch Umsetzung mit Verbindungen der allgemeinen Formel (IV)

$R^4$-N=C=O (IV)

in welcher

$R^4$ die oben angegebene Bedeutung hat,

in inerten Lösemitteln, in Anwesenheit einer Base, in die Verbindungen der allgemeinen Formel (V)

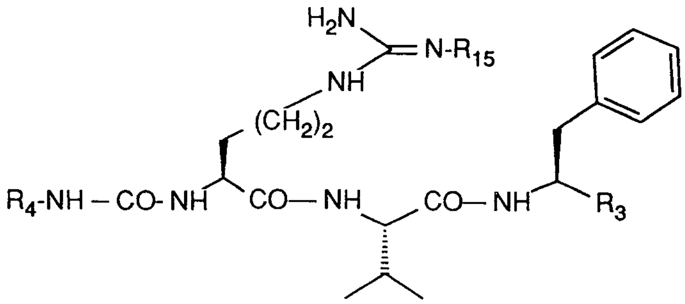

(V)

in welcher

$R^3$, $R^4$ und $R^{15}$ die oben angegebene Bedeutung haben,

überführt,

und im Fall, $R^3$ = $CH_2$-OH, die Verbindungen der allgemeinen Formel ($R^3$ = -$CO_2CH_3$) (V) nach üblichen Methoden, vorzugsweise aber mit Natriumborhydrid umsetzt,

und im Fall, $R^3$ = CHO, die Verbindungen der allgemeinen Formel (V), ausgehend von der Hydroxymethylverbindung ($R^3$ = $CH_2$-OH) einer Swern-Oxidation unterzieht,

in Abhängigkeit des Restes $R^{15}$, beispielsweise mit Fluorwasserstoffsäure oder Trifluoressigsäure umsetzt,

und im Fall einer Aminoschutzgruppe ($R^1$/$R^{15}$) diese nach denen in der Peptidchemie üblichen Methoden abspaltet,

und im Fall der Säuren die Ester verseift.

**5.** Arzneimittel enthaltend eine oder mehrere Verbindungen aus den Ansprüchen 1 bis 3.

**6.** Verwendung der Verbindungen aus den Ansprüchen 1 bis 3 zur Herstellung von Arzneimitteln.

**7.** Verbindungen der allgemeinen Formel (III)

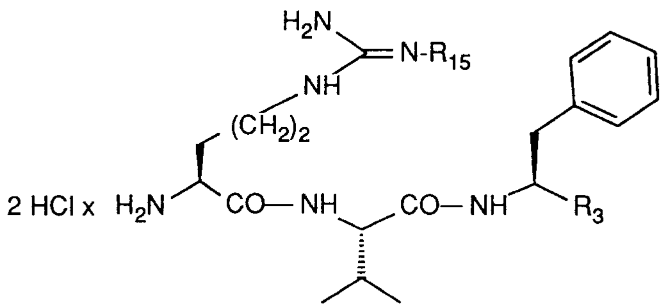

(III)

in welcher

$R^3$ die in den Ansprüchen 1 bis 3 angegebene Bedeutung hat

und

$R^{15}$ die in den Ansprüchen 1 bis 3 angegebenen Bedeutung von $R^2$ hat, aber nicht für Wasserstoff steht.

**8.** Verfahren zur Herstellung der Verbindungen der allgemeinen Formel (III) dadurch gekennzeichnet, daß man Verbindungen der allgemeinen Formel (VI)

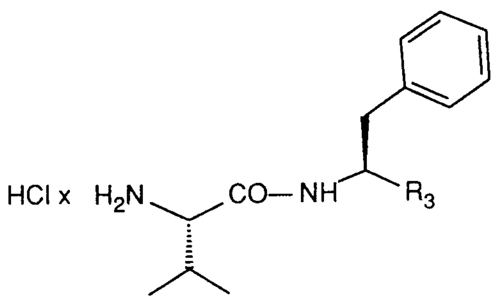

(VI)

in welcher

$R^3$ die in Anspruch 7 angegebene Bedeutung hat,

mit den Aminosäurederivaten der Formel (VII)

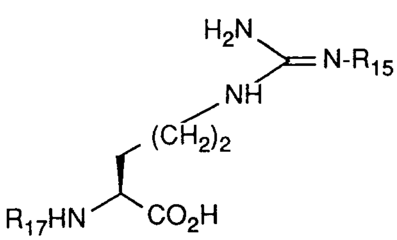

(VII)

in welcher

$R^{15}$ die in Anspruch 7 angegebene Bedeutung hat

und

$R^{17}$ für eine der oben aufgeführten Aminoschutzgruppen, vorzugsweise 9-Fluorenylmethoxycarbonyl (Fmoc), tert.Butoxycarbonyl (Boc) oder Benzyloxycarbonyl (Z) steht, in einem der oben angegebenen Lösemittel, vorzugsweise Methylenchlorid, in Anwesenheit eines Hilfsstoffes und/oder Base, vorzugsweise HOBT und Dicyclohexylcarbodiimid, umsetzt

und anschließend, ebenfalls nach üblichen Methoden, die Aminoschutzgruppe, vorzugsweise mit Salzsäure in Dioxan abspaltet.